# EUROPEAN PATENT APPLICATION

(11) **EP 0 275 101 A2**
(43) Date of publication of application: **20.07.1988**
(21) Application number: 88100452.7
(22) Date of filing: 14.01.1988
(51) Int. Cl.: C07K 5/02, C07C 103/44, C07C 103/82, C07C 125/06, C07K 1/00, A61K 37/64

(54) **Novel peptidase inhibitors**

(30) Priority: 16.01.1987 US 5799; 18.12.1987 US 132262
(71) Applicant: MERRELL DOW PHARMACEUTICALS INC., Cincinnati Ohio 45215-6300 (US)
(72) Inventor: Schirlin, Daniel, F-67800 Bischheim (FR); Jung, Michel, F-67350 Pfaffenhoffen (FR)
(74) Representative: VOSSIUS & PARTNER

(57) **Abstract**

This invention relates to activated electrophillc ketone retroamide analogs of certain peptidase substrates which are useful in inhibiting serine-, thiol-, carboxylic acid- and metallo- dependent proteolytic enzymes, the inhibttion of which will have useful physiological consequences in a variety of disease states.

## Description

### NOVEL PEPTIDASE INHIBITORS

This application is a continuation-in-part of application serial number 005,799, filed January 16,1987.

This invention relates to protease enzyme inhibitors useful for a variety of physiological end-use applications.

In its broad aspect, this invention relates to analogs of peptidase substrates in which the nitrogen atom of the scissile amide bond of a partial retropeptide analog of the substrate has been replaced by a difluoromethylene moiety. These peptidase substrate analogs provide specific enzyme inhibitors for a variety of proteases, the inhibition of which exert valuable pharmacological activities and therefore have useful physiological consequences in a variety of disease states.

In its more specific aspects, this invention relates to activated electrophilic ketone retroamide analogs of certain peptidase substrates which are useful in inhibiting serine-, thiol-, carboxylic acid- and metallo-dependent proteolytic enzymes, the inhibition of which will have useful physiological consequences in a variety of disease states.

Still more specifically, this invention relates to activated electrophilic ketone retroamide analogs of peptidase substrates which fall within the following generic groupings characterized according to their active

site dependencies. Such generic groupings are:
I. Serine Dependent Enzymes: These include such enzymes such as Elastase (human leukocyte), Cathepsin G, Thrombin, Plasmin, C1 Esterase, C-3 Convertase, Urokinase, Plasminogen Activator, Acrosin, p-Lactamase, D-Alanine-D-Alanine Carboxypeptidase, Chymotrypsin, Trypsin and Kallikreins.
II. Thio Dependent Enzymes: Cathepsin B.
III. Carboxylic Acid Dependent Enzymes: These include such specific enzymes as Renin, Pepsin and Cathepsin D.
IV. Metallo Dependent Enzymes: These include Angiotensin Converting Enzyme, Enkephalinase, Pseudomonas Elastase and Leucine Aminopeptidase.

The contemplated peptidase inhibitors of the foregoing enzymes are selected from the generic formula 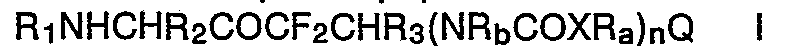 the hydrates, isosteres or the pharmaceutically acceptable salts therof wherein:
R is hydrogen, an amino protecting group selected from Group K, an a-amino acid or a peptide comprised of a number of a-amino acid building blocks, each of said a-amino acid or peptide optionally bearing an amino protecting group preferably selected from Group K,
R₂ is a specific characteristic "R group" side chain of the a-amino acid building block responsible for directing the inhibitor to the active site of the enzyme,
R₃ is H, Ci-6 stright or branched alkyl, phenyl, benzyl, cyclohexyl, cyclohexylmethyl or 2-pyridylmethyl, or is the R-group residue of an a-amino acid building block for that peptidase substrate analog,
n is an integer of 1 to 10,
Ra is a specific characteristic "R-group" side chain of an a-amino acid building block for that peptidase substrate analog, or is an ethylene moiety which when attached to the N atom of that retroamide moiety forms a 2-oxopyrrolidine moiety,
R_{b} is H, Ci -₆ straight or branched alkyl, or an ethylene moiety which when linked to the CH moiety of X forms a 2-oxopyrrolidine moiety,
X is H, CH, OR₇ or R₇ with R₇ being Ci-₆ straight or branched alkyl, phenyl, benzyl, cyclohexyl, cyclohexylmethyl or 2-pyridylmethyl with the proviso that when X is other than CH, Rₐ and Q are deleted,
Q is H, C₁₋₁ o alkyl or aralkyl, CORsY, or COY with Rs being an a-amino acid R-group or a peptide comprised of a-amino acid building blocks, and
Y is NHR₄ or OR₄, with R₄ being H, C₁₋₆ straight or branched alkyl, phenyl, benzyl, cyclohexyl, cyclohexylmethyl or 2-pyridylmethyl.

Isosteres of the compounds of formula I include those wherein (a) one or more of the a-amino residues of the Ri and Q substituents are in their unnatural configuration (when there is a natural configuration) or (b) when the normal peptidic carbamoyl linkage is modified, such as for example to form -CH₂NH- (reduced), -COCH₂- (keto), -CH(OH)CH₂- (hydroxy), -CH(NH₂)CH₂)- (amino), -CH₂CH₂-(hydrocarbon). Preferably a compound of the invention should not be in an isosteric form, particularly it is preferred that there be no modified peptidic carbamoyl group in the Rᵢ and Q radicals, but if there is, it is preferable to keep the isosteric modifications to a minimum.

A compound of the invention may be in free form, e.g., amphoteric form, or in salt, e.g., acid addition or anionic salt, form. A compound in free form may be converted into a salt form in an art-known manner and vice-versa. Examples of salt forms are the trifluoroacetate, hydrochloride, sodium potassium and ammonium forms.

Unless otherwise stated, the a-amino acid building blocks of these peptidase substrate analogs are preferably in their L-configuration. However, in those instances wherein there is an amide peptide bond between the CF₂ and a resulting malonyl moiety, then the a-amino acid building blocks between the CF₂ moiety and the malonyl moiety are in their D-configuration.

In the event n is 2 then there are two Rₐ moieties, (i.e., Rₐi and Rₐ₂, each of which may be the same or different), if n is 3 then there are three Rₐ moieties, (i.e., Rai, Rₐ₂ and Rₐa) each of which may be the same or different and so on for each change in the definition of n. Analogously, the X and R_{b} moieties would similarly be increased as n is increased, each being modified Independently within the scope of their generic definitions.

Before further defining and/or illustrating the scope of the peptidase inhibitors embraced by formula I, it may be convenient to state some of the more basic concepts related to peptldes. For example, except for proline, all of the a-amino acids found In proteins have as a common denominator a free carboxyl group and a free unsubstituted amino group on the a-carbon atom (in proline, since proline's a-amino group is substituted it is really an a-amino acid, but for convenience, it will also be spoken of as an a-amino acid). Additionally, each a-amino acid has a characteristic "R-group", the R-grdup being the side chain, or residue, atached to the a-carbon atom of the a-amino acid. For example, the R-group side chain for glycine is hydrogen, for alanine it is methyl, for valine it would be isopropyl. (Thus, throughout this specification the R ₁, R₂, R₃ or R₅ moiety is the R-group for each indicated a-amino acid). For the specific R-groups - or side chains - of the a-amino acids reference to A.L. Lehninger's text on Biochemistry (see particularly Chapter 4) would be helpful. In those instances wherein Ra is an ethylene moiety attached to the CH group of X and to the nitrogen atom of that retroamide, that resulting 2-oxopyrrolidine moiety is represented by 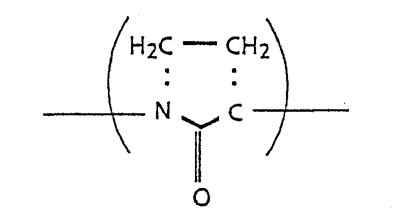 wherein the dotted lines depict the ethylene moiety attached to the nitrogen atom of the retroamide.

As a further convenience for defining the scope of the compounds embraced by the generic concept of formula I, as well as the sub-generic concepts relating to each of the individual enzymes Involved in this invention, various a-amino acids have been classified into a variety of groups which impart similar functional characteristics for each of the specific enzymes to be inhibited by the peptidase substrates of formula I. These groups are set forth in Table Il and the recognized abbreviations for the a-amino acid blocks are set forth In Table I.

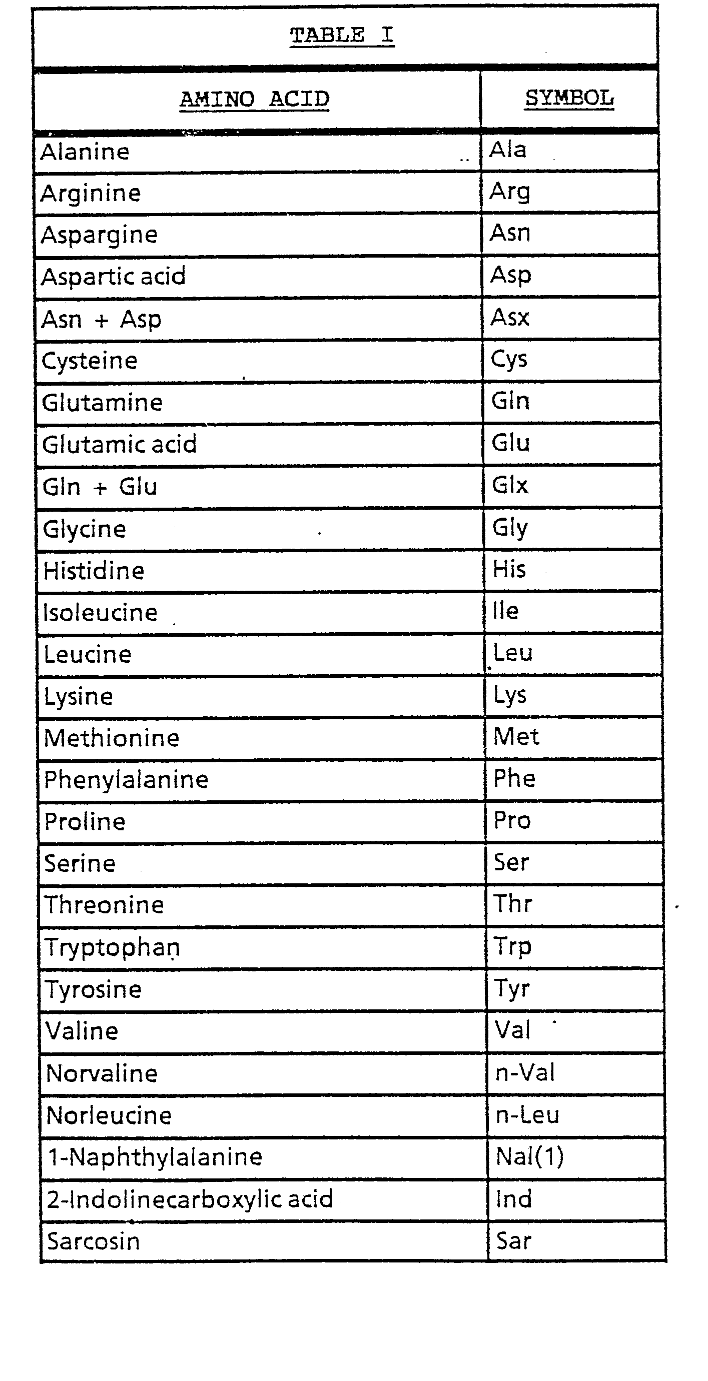

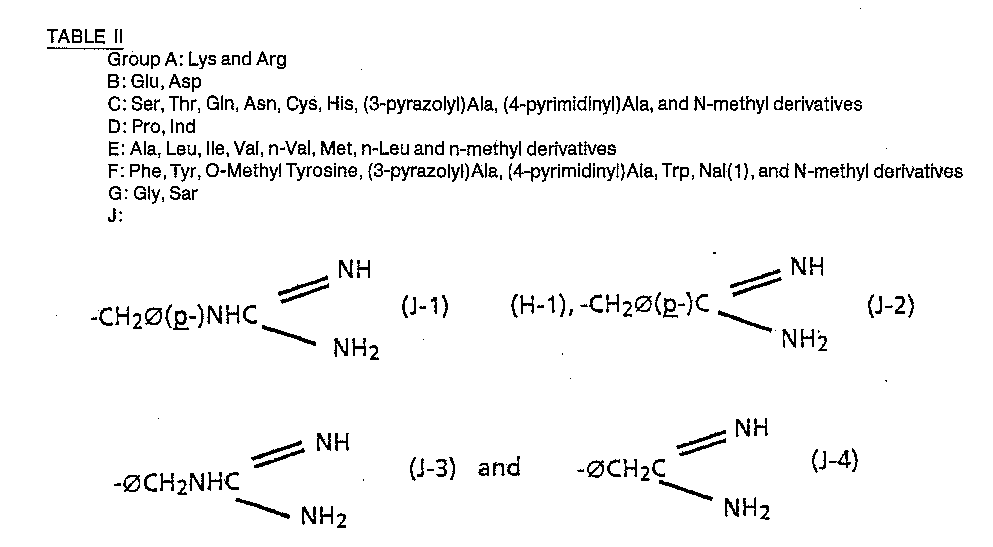

with 0, of course, represeting phenyl (it being understood that the bond of J1-4 is always attached to an amino acid)

K: Acetyl (Ac), Succinyl (Suc), Benzoyl (Bz), t-Butyloxycarbonyl (Boc), Carbobenzoxy (CBZ), Tosyl (Ts), Dansyl (DNS), lsovaleryl (Iva), Methoxysuccinyl (MeOSuc), 1-Adamantanesulphonyl (AdS0₂), 1-Adamantaneacetyl (AdAc), 2-Carboxybenzoyl (2-CBZ), Phenylacetyl, t-Butylacetyl (Tba) or bis [(1-naphthyl)methyl]acetyl (BNMA) and such other terminal amino protecting groups which are functionally equivalent thereto

In light of the foregoing, the defined compounds of formula I may also be stated as being:

An activated electrophilic ketone-bearing peptidase inhibitor of the formula 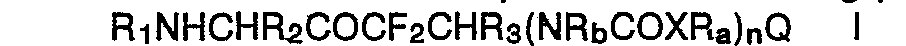 the hydrates, isosteres or the pharmaceutically acceptable salts thereof wherein
R₁ is hydrogen, an amino protecting group selected from Group K, an a-amino acid or a peptide comprised of a number of a-amino acid building blocks, each of said a-amino acid or peptide optionally bearing an amino protecting group preferably selected from Group K,
R₂ is a specific characteristic "R group" side chain of the a-amino acid building block responsible for directing the inhibitor to the active site of the enzyme,
R₃ is H, Ci-6 straight or branched alkyl, phenyl, benzyl, cyclohexyl, cyclohexylmethyl or 2-pyridyl-methyl, or is the R-group residue of an a-amino acid building block for that peptidase substrate analog,
n is an integer of 1 to 10,
Rₐ is a specific characteristic "R-group" side chain of an a-amino acid building block for that peptidase substrate analog, or is an ethylene moiety which when attached to the N atom of that retroamide moiety forms a 2-oxopyrrolidine moiety,
R_{b} is H, C₁₋₆ straight or branched alkyl, or an ethylene moiety which when linked to the CH moiety of X forms a 2-oxopyrrolidine moiety,
X is H, CH, OR₇ or R₇ with R₇ being Cₗ-₆ straight or branched alkyl, phenyl, benzyl, cyclohexyl, cyclohexylmethyl or 2-pyridylmethyl with the proviso that when X is other than CH, Rₐ and Q are deleted, Q is H, C₁₋₁ o alkyl or aralkyl, CORsY, or COY with Rs being an a-amino acid R-group or a peptide comprised of a-amino acid building blocks,
Y is NHR₄ or OR₄, with R₄ being H, C₁₋₆ straight or branched alkyl, phenyl, benzyl, cyclohexyl, cyclohexylmethyl or 2-pyrldylmethyl, wherein the said a-amino acid and peptide noleties are building blocks selected from Groups A, B, C, D, E, F, G and J, and K is a terminal amino protecting group, members of these groups being 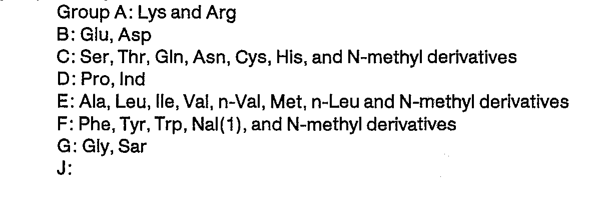 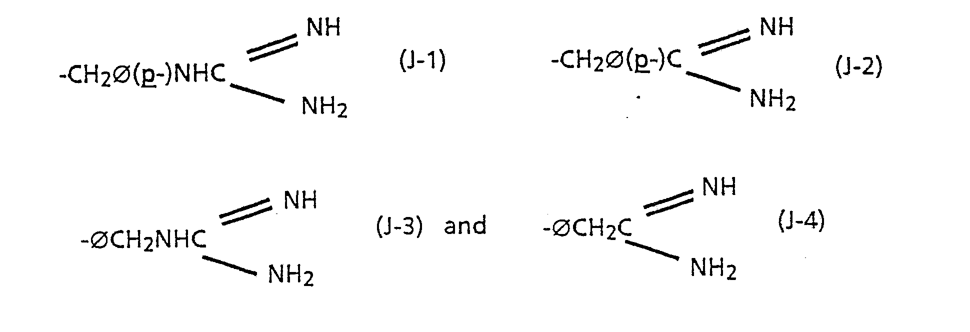 with 0, of course, representing phenyl (it being understood that the bond of J1-4 is always attached to an amino acid).

K: Acetyl (Ac), Succinyl (suc), Benzoyl (Bz), t-Butyloxy-carbonyl (Boc), Carbobenzoxy (CBZ), Tosyl (Ts), Dansyl (DNS), Isovaleryl (Iva), Methoxysuccinyl (MeOSuc), 1-Adamantanesulphonyl (AdS0₂), 1-Adamantaneacetyl (AdAc), 2-Carboxybenzol (2-CBZ), Phenylacetyl, t-Butylacetyl (Tba) or bis [(1-naphthyl)methylacetyl (BNMA) and such other terminal amino protecting groups which are functionally equivalent thereto.

An alternate expressive of structural formula I is depicted formula II as follows: 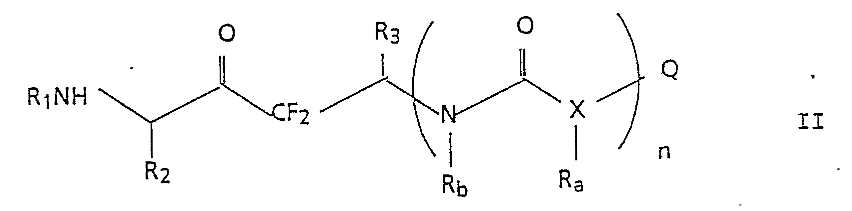 wherein Ri, R₂, R₃, R_{b}, Ra, n and Q are as defined for formula I. In this depiction the R₂ moiety is in the Pi position of the peptide, the a-amino acids of the Ri moiety would be in the P₂→Pₙ positions, n being the numeric sequence dependent upon the number of a-amino acid building blocks in that particular compound, e.g., if R₁ contained four a-amino acids it would be comprised of P₂-P₃-P₄-P₅ positions with the option of a terminal amino protecting group from Group K in the P₅ moiety. The moiety containing CF₂ and R₃ is in the Pi' position of the peptide.

Formula III is used to further illustrate the type compounds embraced by formula I and to more specifically illustrate the shorthand method of naming these compounds, as follows:

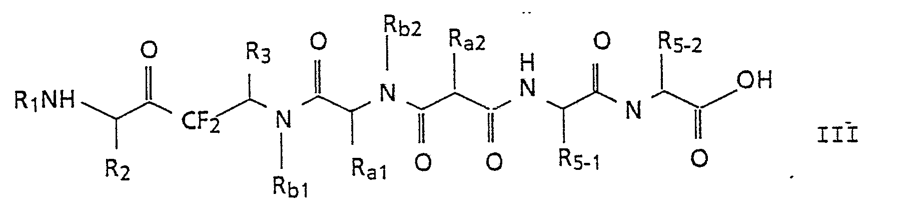

This formula illustrates a compound containing two retroamide moieties, a malonyl moiety, N-substitutions for R_{b} moieties, X being CH, n being 2, and Q being CORsY with Rₛ being a dipeptide moiety and Y being OH, which depiction may be written in accepted shorthand nomenclature as R₁-R₂[CF₂R₃NR_{b-} 1](N-R_{b2}-Rₐ₁)-m-Rₐ₂-R₅₋₁-R₅₋₂OH.

From formula III it is quite obvious that the bracketed CF₂-retroamide inhibiting moiety is a moiety wherein the nitrogen atom of the a-amino acid has been replaced by CF₂ radical, the R-residue remaining as defined for R₃ and the amide bond linking the two amino acids corresponding to the P'i and P'₂ positions has been reversed. Similarly, the amide bond linking the P'₂ and P's positions has also been reversed. The brackets designate the retroamide moiety containing the CF₂ moiety, the parenthesis embracing the NR_{b2}-Rₐₗ moiety indicates that it also is in a retroamide configuration and the underlined m (i.e., meta) indicates a malonyl moiety containing the Rₐ-₂ a-amino acid residue.

To further illustrate the shorthand nomenclature used throughout this application assume that Ri is comprised of P₂, P₃, P₄ having a terminal amino protecting group so that Ri is -Pro-Ala-Ala-Suc-OCH₃, R2 is Val, Rs is Gly, R_{b1} is ethyl, Rₐ₁ is H, R_{b2} is ethyl, Rₐ₂ is Gly, Rs-i is Val, R₅₋₂is Gly, then that specific compound would be written as H₃CO-Suc-Ala-Ala-Pro-Val;-[CF₂GlyN-Et](N-EtGly)-m-Gly-Val-GlyOH.

It is also to be noted that in some instances it is more convenient to designate the terminal amino protecting group as a separate Pₙ position of the peptide, for example, in illustrative formula III. The terminal amino protecting group would be designated as being in the P₅ position and thus R₁ would be P₂-P₃-P₄-P₅ with Ps being a protecting group of Group K. If P₄ optionally is an R group or is delected, then quite obviously, when P₄ is delected the protecting group of P₅ would be attached to the P₃ moiety.

In general and unless otherwise stated, from the CF₂ inhibiting moiety to the malonyl moiety the amino acids (or R Groups) are in their D-configuration; thereon they are in their L-configuration.

Utilizing the foregoing illustrations those compounds of formula I which are useful as inhibitors for human leukocyte elastase are represented by the formula
Ri NHCHR₂COCF₂CHR₃(NR_{b}COXRₐ)ₙQ la
and the hydrates, isosteres or the pharmaceutically acceptable salts thereof, wherein
Ri is -P₂P₃P₄P₅ with
R₂ being a-amino acid block having an R group selected from Groups D, E and F, with proline being preferred, P₃ is the a-amino acid blocks of Groups D, E, or lysine with isoleucine, valine or alanine being preferred, P₄ is the a-amino acid blocks of Groups E or zero with alanine being preferred (when Pn Is zero then that particular moiety does not appear in the structure, I.e., it is deleted), and
Pₛ is a terminal moiety of Group K with methoxysuccinyl and CBZ being preferred,
R₂ is the R Group of Groups E and G, with nor-valine and valine being preferred,
R₃ is an R Group selected from Groups E and G, with glycine and alanine being preferred,
Rₐ is an R Group selected from Groups E and G or is phenyl, with alanine, phenyl and glycine being preferred, with R_{b}, X, n and Q being as generically defined for formula I, n preferably being 1 or 2, Rbpreferably being H, X preferably being CH, Q preferably being H or CONH₂ and R₅, when present, is an a-amino acid having an R group selected from Groups E and G with alanine being preferred and then Y preferably is NH₂.

Human leukocyte elastase is released by polymorphonuclear leukocytes at sites of inflammation and thus is a contributing cause for a number of disease states. Thus the peptidase substrates of formula (la) have an anti-inflammatory effect useful in the treatment of.gout, rheumatoid arthritis and other inflammatory diseases, and in the treatment of emphysema. In their end-use application the enzyme inhibitory properties of the compounds of (la) are readily ascertained by standard biochemical techniques well known in the art. Potential dose range for their end-use application will of course depend upon the nature and severity of the disease state as determined by the attending diagnostician with the range of 0.01 to 10 mg/kg body weight per day being useful for the aforementioned disease states with 0.1 mg to 10 mg/kg per day being preferred. The preferred compounds for this enzyme are:

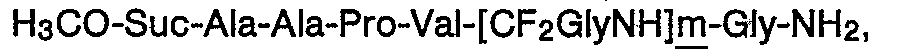

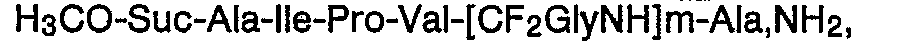

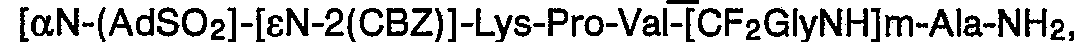

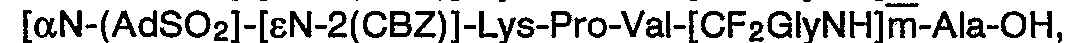

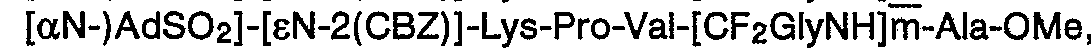

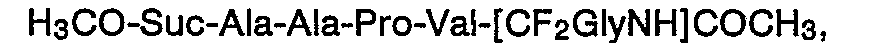

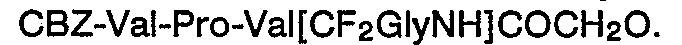

Those compounds of formula I which are useful as inhibitors of Cathepsin G are represented by the structural formula
RiNHCHR₂COCF₂CHR₂(NR_{b}COXRa)nQ Ib
   and the hydrates, isosteres or the pharmaceutically acceptable salts thereof, wherein
Ri is -P₂P₃P₄P₅ with P₂ being selected from Groups D, E, or G, with proline being preferred
Ps is selected from Groups E or G with alanine being preferred,
P₄ is selected from Groups E, G or is deleted with alanine being preferred, the terminal a-amino acid optionally bearing a protecting group selected from Group K with succinyl or methoxy succinyl being preferred, R₂ is selected from Groups E and F but preferably is Phe,
Rs is as generally defined for formula I with the -amino acid residue being selected from Groups E and G, Gly, Ala and Phe being preferred,
R_{b}, X, n and Q are as generically defined in formula I with R_{b} preferably being H, n preferably being 1, Q preferably is H and Rₐ and R₅ are as defined for formula (la).

The end-use application of the compounds (lb) inhibiting Cathepsin G is the same as for human leukocyte inhibitors, including arthritis, gout and emphysema, but also embracing the treatment of glomerulonephritis and lung infestations caused by infections in the lungs. For their end-use application, the potency and other biochemical parameters of the enzyme inhibiting characteristics of the compounds of (lb) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 to 10 mg/kg per day being preferred. Preferred compounds for formula (lb) are:

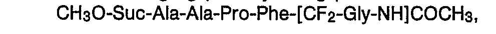

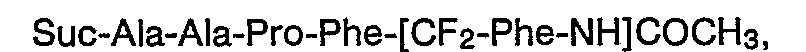

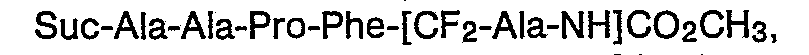

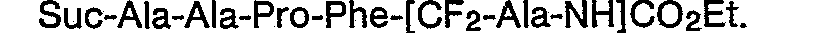

Those compounds of formula I which are useful as inhibitors of thrombin are represented by the formula R₁NHCHR₂COCF₂CHR₃(NR_{b}COXRₐ)ₙQ 1c
and the hydrates, isosteres or the pharmaceutically acceptable salts thereof, wherein
Ri is (a) -P₂-Ps, (b) -P₂ or (c) -P₂-P₃-P₄ wherein (a) P₂ is selected from Groups D, E or F, preferably proline, P₃ is selected from Group F, each Ps is selected from Group F, each P3 being inthe D-configuration, preferably D-Phe,
   (b) P₂ is selected from Group K but preferably is dansyl, tosyl or benzoyl,
   (c) P₂ is selected from Group E but preferably is alanine, P₃ is selected from Groups C, "G and E but preferably is serine, P₄ is selected from Groups F, G and E or is zero but preferably is Phe,
R₂ is preferably the arginine side chain but may also be selected from Groups A and J, preferably JI, R₃ is as generally defined for formula I, with the residue being selected from Groups C and G with glycine and serine being preferred,
Rₐ is selected from Group C or G but preferably is a glycine or serine side chain,
R_{b}, n, X and Q are as generically defined tor formula I, R_{b} preferably being H, X preferably being CH, and Q preferably is H or alkyl and
Rs is preferably the glycine amino acid residue or is a residue of an a-amino acid of Group E or D or is zero.

The compounds embraced by formula (Ic) inhibit thrombin and therefore, as in the use of heparin, the compounds may be used as the initial anticoagulant agent in thrombophlebitis and coronary thrombosis. For their end-use application, the potency and other biochemical parameters of the enzyme inhibiting characteristics of the compounds of (Ic) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred. Preferred compounds are as expressed for Cathepsin G and also include:

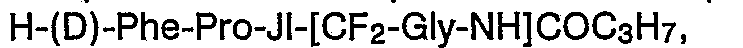

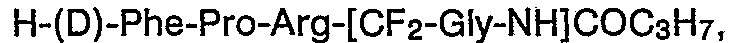

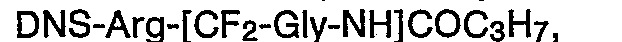

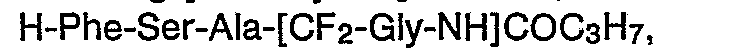

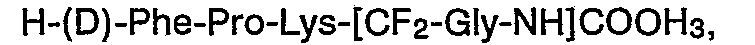

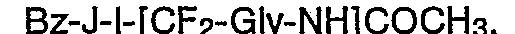

The compounds of formula I which are useful as inhibitors of chymotrypsin are represented by the structural formula
RiNHCHR₂COCF₂CHR₃(NR_{b}COXRₐ)ₙQ Id
and the hydrates, isosteres or the pharmaceutically acceptable salts thereof, wherein
R₃, Ra, R_{b}, X and Q are defined for compounds of (la), and Ri is -P₂P₃P₄P₅ with
P₂ being selected from Groups D, E, with Leu being preferred, G or K with benzoyl being preferred,
P₃ is selected from Groups E or G or K, with acetyl being preferred, or is deleted, with alanine being preferred,
P₄ is selected from Groups E or G or K or is deleted, with alanine being preferred,
P₅ is selected from Group K with succinyl being preferred or is deleted, and
R₂ is selected from Groups E and F but preferably is Phe or Tyr.

The end-use application of the compounds (Id) inhibiting chymotrypsin is int he treatment of pancreatitis. For their end-use application, the potency and other biochemical parameters of the enzyme inhibiting characteristics of the compounds of (Id) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred. Preferred compounds are as expressed for Cathepsin G and also include:

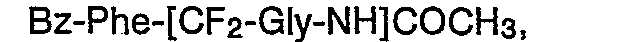

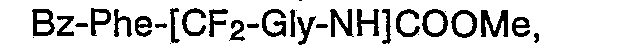

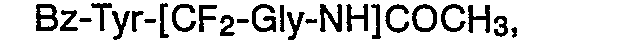

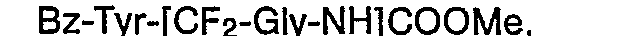

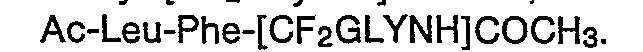

The compounds of formula I which are useful as inhibitors of trypsin are represented b y the structural formula
RiNHCHR₂COCF₂CHR₃(NR_{b}COXRₐ)ₙQ le
   and the hydrates, isosteres or the pharmaceutically acceptable salts thereof, wherein
R₂, R₃, Ra, R_{b}, n, X and Q generally are as defined in (Ic),
R₁ is selected from (a) -P₂-P₃, (b) -P₂ or (c) -P₂-P₃-P₄ with
   (a) P₂ is selected from Groups "E or F but is preferably proline or alanine, P₃ is selected from Group F, (each being in the D configuration) but preferably is (d) -Phe,
   (b) P₂ is selected from Group K but preferably is dansyl, tosyl or benzoyl,
   (c) P₂ is selected from Group D or E but preferably is proline or alanine, P₃ is selected from Groups G and E but preferably is serine, P₄ is selected from Groups G and E or is zero but preferably is Phe.

The end-use application of the compounds (Ie) inhibiting trypsin is in the treatment of pancreatitis. For their end-use application, the potency and other biochemical parameters of the enzyme inhibiting characteristics of the compounds of (le) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg ot 10 mg/kg per day being preferred. The preferred compounds useful for inhibiting trypsin are the same as for the inhibitors of thrombin.

The compounds of formula I which are useful as inhibitors of plasmin are represented by the structuai formula 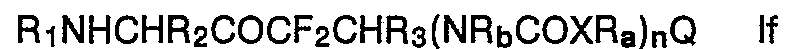 and the hydrates, isosteres or the pharmaceutically acceptable salts thereof, wherein Rs, Rₐ, R_{b}, X, n and Q are as defined in formula I:
Ri is -P₂P₃P₄ with
P₂ being selected from Group E or F but preferably is Ala or Phe,
P₃ is selected from Group B, F or K but preferably is Glu or acetyl, and
P₄ is selected from Group K or is deleted but preferably is dansyl,
R₂ is selected from Groups A and J but preferably is lysine or J-I, and
n preferably is one and
R_{b} preferably is H.

The compounds embraced by formula (If) inhibit plasmin and are therefore antiproliferative agents useful in treating excessive cell growth, particularly in the treatment of benign prostatic hypertrophy and prostatic carcinoma, and in the treatment of psoriasis. For their end-use application, the potency and other biochemical parameters of the enzyme inhibiting characteristics of the compounds of (If) are readily ascertained by standard biochemical techniques well known in the art. Actual dose ranges for their specific end-use application will, of course, depend upon the nature and severity of the disease state of the patient or animal to be treated as determined by the attending diagnostician. It Is to be expected that the general end-use application dose range will be about 0.01 to 10 mg/kg per day for an effective therapeutic effect with 0.1 to 10 mg/kg per day being preferred. The preferred compounds are:

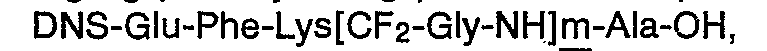

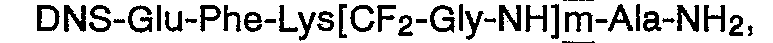

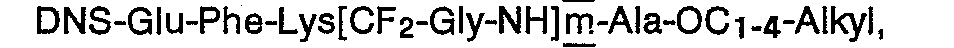

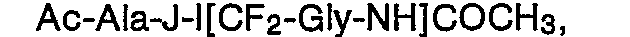

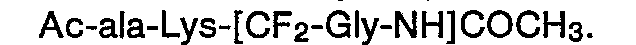

The compounds of formula I which are useful as inhibitors of Ci-esterase are represented by the structural formula
RiNHCHR2COCF2CHR3(NRbCOXRa)nQ Ig
and the hydrates, isoteres or the pharmaceutically acceptable salts thereof wherein
Q, Rs, R_{b} and X are defined in formula I, with the R-group of R₃ selected from Group E or G,
Ri generically is -P₂-P₃ with P₂ being selected from Groups E, G, D, C, F, A or B with Ala being preferred, and
P₃ is selected from Group K with CBZ or acetyl being preferred,
R₂ is selected from Groups A and J, but preferably is Arg or J-I,
Rₐ is selected from Group E or G, preferably Gly,
n preferably is one, and
R_{b} preferably is H.

The compounds embraced by formula (Ig) inhibit Ci-esterase and are therefore useful in treating systemic lupus, arthirits, autoimmune hemolytic anemia and glomerulonephritis. For their end-use application, the potency and other biochemical parameters of the enzyme inhibiting characteristics of the compounds of (Ig) is readily ascertained by standard biochemical techniques well known in the art. Actual dose ranges for their specific end-use application will, of course, depend upon the nature and severity of the disease state of the patient or animal to be treated as determined by the attending diagnostician. It Is to be expected that the general end-use application dose range will be about 0.01 to 10 mg/kg per day for an effective therapeutic effect with 0.1 mg to 10 mg/kg per day being preferred. The preferred compounds are:

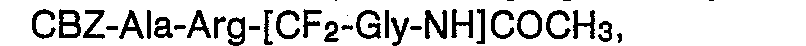

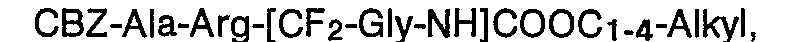

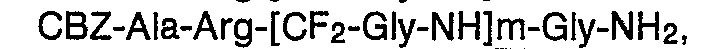

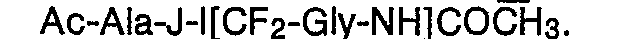

The compounds of formula I which are useful as inhibitors of Cs-convertase are represented by the formula RiNHCHR₂COCF₂CHR₃(NR_{b}COXRₐ)ₙQ Ih
and the hydrates, isosteres or the pharmaceutically acceptable salts thereof, wherein generically
R_{b}, X, n and Q are as defined in formula I,
Rᵢ is -P₂P₃P₄ with
P₂ being selected from Groups E or F, with Ala being preferred,
P₃ is selected from Groups E or F with Leu being preferred, and
P₄ is selected from Group K with Bz being preferred,
R₂ is selected from Grtoups A or J, with Arg being preferred,
R₃ is selected from Groups E or G, with Gly being preferred,
R₅ is zero and
Y preferably is NH₂,
Rₐ is selected from Group "E but preferably is Ala,
R_{b} preferably is H.

The compounds embraced by formula (Ih) inhibit Cₛ-convertase and are therefore useful in treating systemic lupus, arthritis, autoimune hemolytic anemia and glomerulonephratis. For their end-use application, the potency and other biochemical parameters of the enzyme inhibiting characteristics of the compounds of (lh) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred. The preferred compounds are:

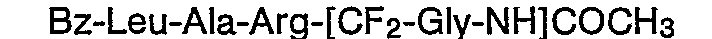

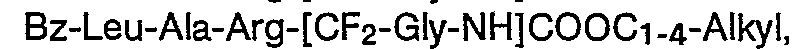

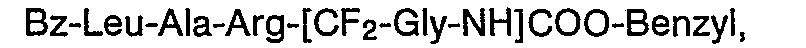

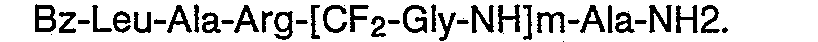

The compounds of formula I which are useful as inhibitors of Urokinase are represented by the formula 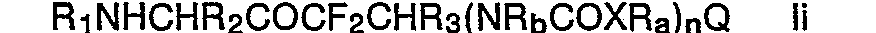 and the hydrates, isosteres or the pharmaceutically acceptable salts thereof, wherein
R₃, Rₐ, R_{b}, X, n and Q are as defined in formula I,
Ri generically is -P₂P₃ with
P₂ being selected from Groups E and G with Ala and Gly being preferred, and
P₃ is selected from Group B with Glu being preferred,
R₂ is selected from Groups A and J with Arg being preferred, and the R-group of R₃ and Rₐ being selected from Group E, each preferably being Ala and
Y preferably being NH₂, and
R_{b} preferably is H.

The compounds embraced by formula (li) inhibit Urokinase and therefore are useful in treating excessive cell growth disease states. As such compounds are useful in the treatment of benign prostatic hypertrophy and prostatic carcinoma, the treatment of psoriasis, and in their use as abortifacients. For their end-use application, the potency and other biochemical parameters of the enzyme inhibiting characteristics of the compounds of (fi) are readily ascertained by standard biochemical techniques well known in the art. Actual dose ranges for their specific end-use application will, of course, depend upon the nature and severity of the disease state, of the patient or animal to be treated as determined by the attending diagnostician. It is to be expected that the general end-use application dose range will be about 0.01 to 10 mg/kg per day for an effective therapeutic effect with 0.1mg to 10 mg/kg per day being preferred. The preferred compounds are:

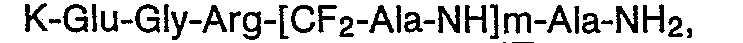

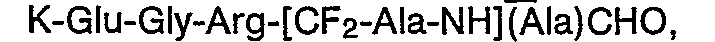

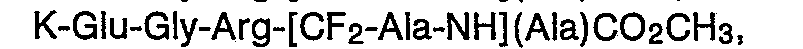

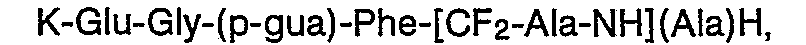

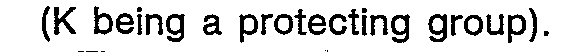

The compounds of formula I which are useful as inhibitors of plasminogen activator are represented by the structural formula 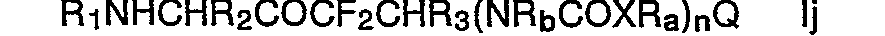 and the hydrates, isosteres or the pharmaceutically acceptable salts thereof, wherein
Rs, Rₐ, R_{b}, X, n and Q are as defined in formula I,
Ri generically is -P₂P₃P₄ wherein
P₂ is Gly,
P₃ is selected from Group B with Glu being preferred, and
P₄ preferably is dansyl but also selected from Group K, and
R₂ is selected from Groups A and J with Arg b eing preferred.

Preferred compounds are:

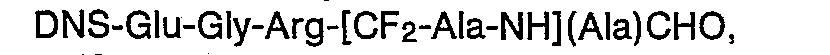

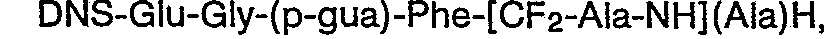

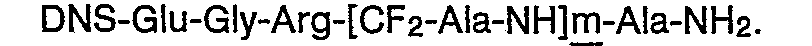

The compounds embraced by formula (lj) inhibit plasminogen activator and therefore are useful in treating excessive cell growth disease states such as, for example, being useful in the treatment of benign prostatic hypertrophy and prostatic carcinoma, in the treatment of psoriasis and in their use as abortifacients. For their end-use application, the potency and other biochemical parameters of the enzyme inhibiting characteristics of the compounds of (lj) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of acrosin are represented by the structural formula 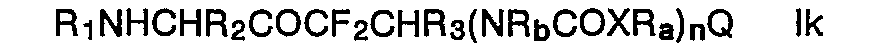 dose range will be about 0.01 to 10 mg/kg per day for an effective therapeutic effect with 0.1 mg to 10 mg/kg per day being preferred.

The compounds of formula I which are useful as inhibitors of β-lactamase are represented by the structural formula 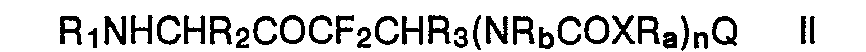 and the hydrates, isosteres or the pharmaceutically acceptable salts thereof, with the proviso that the Pi carbonyl moiety may exist in its chemically reduced form i.e., R₁NHCHR₂ wherein 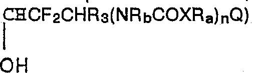
R₁ is P₂,
P₂ being selected from Group K with COCh₂0 and Bz being preferred,
R₂ is selected from Groups E, G and C with glycine being preferred, and
Rₛ, Rₐ, R_{b}, X, n and Q being as defined in formula I, with the R groups of R₃ being selected from Group E or G, and n preferably is one.

The preferred compounds are:

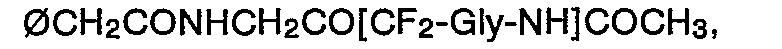

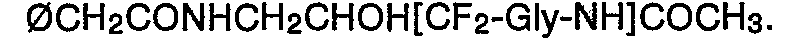

The compounds embraced by formula (II) inhibit p-lactamase and therefore are useful in the potentiation of antibacterial agents, particularly the β-lactam antibacterials. For their end-use application, the potency and other biochemical parameters of the enzyme inhibiting characteristics of the compounds of (II) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of D-Ala-D-Ala carboxypeptidase are represented by the structural formula 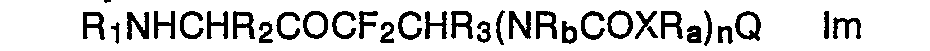 and the hydrates, isosteres or the pharmaceutically acceptable salts thereof, wherein
R₁ is P₂-P₃ with
P₂ being Ns-Ac-Lys or is selected from Groups E and C with Ns-Ac-Lys being preferred,
P₃ is selected from Group K with Ac being preferred,
R₂ is D-Ala, and
R₃, Ra, R_{b}, X and Q are as defined in formula I, with the R-group of Rs being selected from Group E with D-Ala being preferred,
n preferably is one,
R_{b} being preferably H,
Rs preferably is deleted and
Y preferably is OH.

The preferred compounds are:

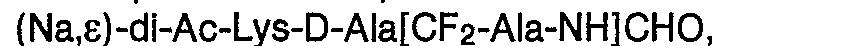

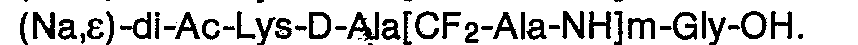

The compounds embraced by formula (lm) are antibacterial agents particularly useful against gram negative organisms. For their end-use application, the potency and other biochemical parameters of the enzyme inhibiting characteristics of the compounds of (Im) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of Cathepsin B are represented by the structural formula 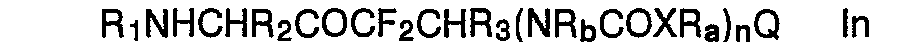 and the hydrates, isosteres or the pharmaceutically acceptable salts thereof, wherein Ri is (a) -P₂-P₃ or (b) -P₂-P₃-P₄ wherein
(a) P₂ is selected from Groups E and F with Phe being preferred and,
   P₃ is selected from Group K with CBZ being preferred,
(b) P₂ is selected from Groups E and F with Leu being preferred,
^{D}₃ being selected from Groups E and F with Leu being preferred, and
R₄ is selected from Group K with Ac being preferred,
R₂ is selected from Groups A and J or ThrOCH₂0, with Arg being preferred, and
R₃, Ra, R_{b}, X, n and Q are as defined in formula I.

The preferred compounds are:

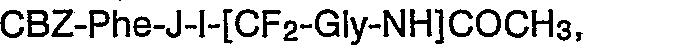

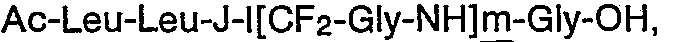

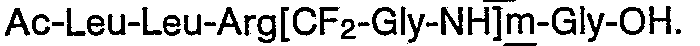

The compounds embraced by formula (In) inhibit Cathepsin B and therefore are useful in treating excessive cell growth disease states such as, for example, being useful in treating benign prostate hypertrophy, prostatic carcinoma, in treating psoriasis and in their use as abortifacients. For their end-use application, the potency and other biochemical parameters of the enzyme inhibiting characteristics of the compounds of (In) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of renin are represented by the structural formula 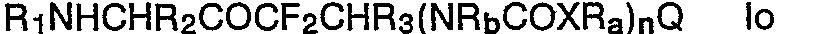 and the hydrates, isosteres or the pharmaceutically acceptable salts thereof, with the proviso that the carbonyl moiety of P-1 may exist in its chemically reduced form, (i.e., R₁NHCHR₂CHOHCF₂CHR₃)NR_{b}COXRₐ)ₙQ) wherein
Ri generically is -P₂P₃P₄P₅P₆ wherein
P₂ is selected from Groups E, C or F with His, n-Val, (3-pyrazolyl)Ala or (4-pyrimidinyl)Ala, and n-Leu being preferred,
P₃ is selected from Groups E or F or is deleted with Phe or O-Methyl Tyrosine being preferred,
P₄ is selected from Groups E, D, F or is deleted with Pro being preferred,
P₅ is selected from Groups E, C, F or is deleted with His being preferred, and
P₆ is selected from Group K with Boc, CBZ or Tba being preferred, or being BNMA when Ps, P₄, P₅ are deleted.
R₂ is selected from Groups E or F or is cyclohexylmethylene with Leu or cyclohexylmethylene being preferred, and
Rs, Rₐ, R_{b}, X, n and Q are defined in formula I with
Ra preferably being selected from Groups G, E, or F,
R_{b} preferably being H,
X preferably being CH, and
Rs is (a) Px' or (b) Px'-P'x+i (x being the appropriate number for that particular p' position of any given particular compound with
Px' being selected from Groups E, C or F or is deleted, with His being preferred,
P'x+i being selected from Groups E or C or is deleted, or is Lys with Lys being preferred, and
Y preferably is OH, or NHR₄ with R₄ preferably being H.

The preferred compounds are: 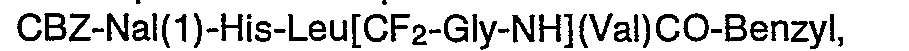 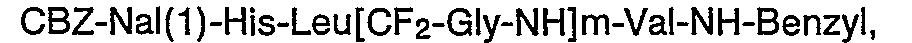 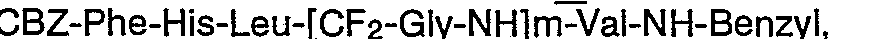 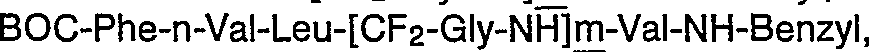 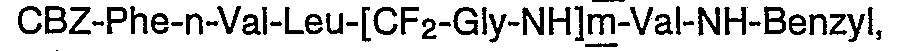 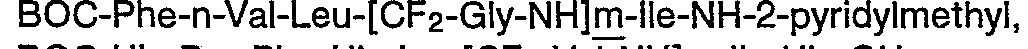 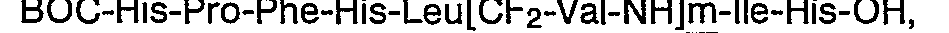 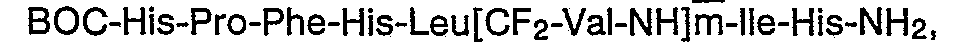 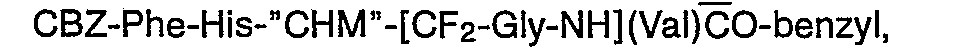 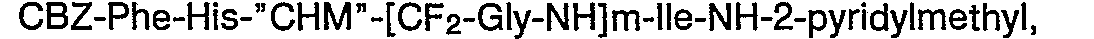 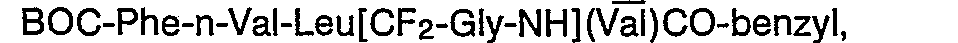 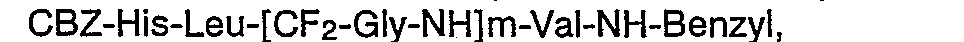 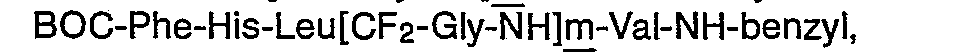 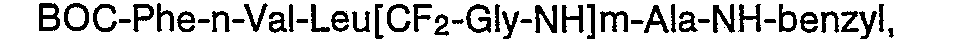 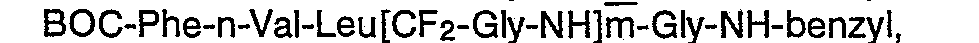 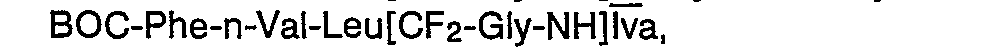 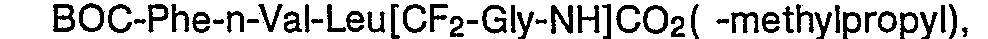 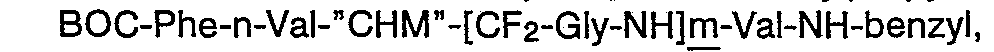 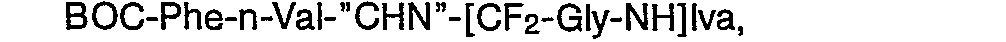 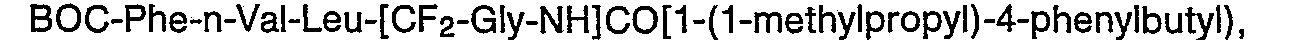 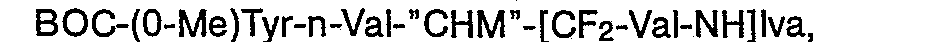 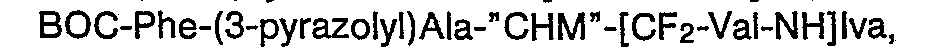 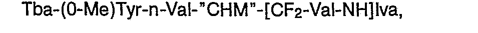 Tba-(O-Me)Tyr-(4-pyrimidinyl)Ala-"CHM"-[CF₂-Val-N H]Iva, with "CHM" being an abbreviation for cyclohexylmethylene.

The compounds of formula (lo) inhibit renin and therefore are used as antihypertensive agents useful in treating hypertension. For their end-use application, the potency and other biachemcial parameters of the enzyme inhibiting characteristics of the compounds of (lo) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with .1 mg to 10 mg/kg per day being preferred.

The compounds of formula I which are useful as inhibitors of pepsin are represented by the structural formula 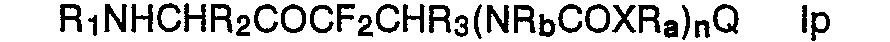 and the hydrates, isosteres or the pharmaceutically acceptable salts thereof, with the proviso that the P-1 carbonyl moiety may exist in its chemically reduced form (i.e., RiNHCHR₂CHOHCF₂CHR₃(NR_{b}COXRₐ)ₙQ), wherein
Rs, Ra, R_{b}, X, n and Q are as defined in formula I,
Ri is -P₂P₃P₄ with
P₂ being selected from groups E or F with Val being preferred,
P₃ is selected from Groups E or F with Val being preferred or is deleted and
P₄ is selected from Group K, preferably Iva, and
R₄ is selected from the Groups E and F with Leu being preferred,
Y is preferably -NH(CH₂)₂CH(CH₃)₂(laa) or -NHCH₂(CH₃)₂ (with (laa) being isoamylamide) and
R₃ preferably is Gly.

The preferred compounds are:

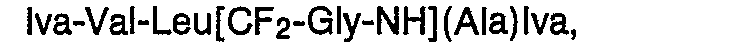

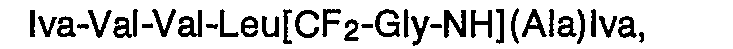

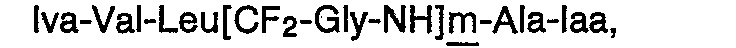

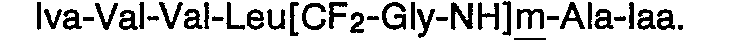

The compounds of formula (Ip) inhibit pepsin and therefore exert an antiulcer effect useful in the treatment and prevention of ulcers. For their end-use application, the potency and other biochemical parameters of the enzyme inhibiting characteristics of the compounds of (Ip) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of Cathepsin D are represented by the structural formula 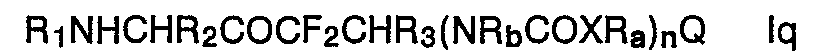 and the hydrates, isosteres or the pharmaceutically acceptable salts thereof, wherein
Rs, Ra, R_{b}, n, X and Q are as defined in formula I,
Ri generically is -P₂P₃P₄ with
P₂ being selected from Groups E and F, with Val or Ala being preferred,
P₃ is selected from Groups E and F or is deleted with Val being preferred, and
P4 is selected from Group K with CBZ being preferred, and
R₂ is selected from Groups E and F, with Phe being preferred,
Y preferably is -NH(CH₂)₂CH(CH₃)₂(laa) or -NHCH₂CH(CHs)₂,
X preferably is CH,
Ra preferably is Ala,
n preferably is 1 or 2,
Q preferably is H or COY or CORsY with
Rs preferably being Phe.

The preferred compounds are:

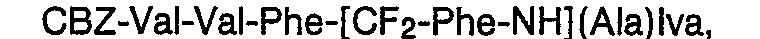

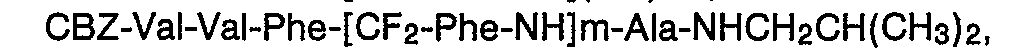

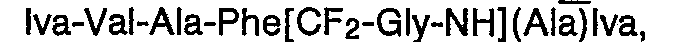

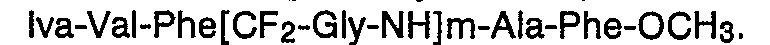

As inhibitors of Cathepsin D the compounds of formula (lq) are useful for the same end-use applications set forth for human leukocyte elastase inhibitors (la) and are also useful as antidemyelinating agents useful to prevent and arrest nerve tissue damage. For their end-use application, the potency and other biochemical parameters of the enzyme inhibiting characteristics of the compounds of (lq) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of angiotensin converting enzyme (ACE) are represented by the structural formula 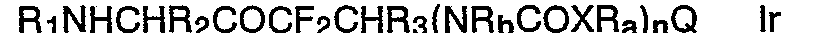 and the hydrates, isoteres or the pharmaceutically acceptable salts thereof, wherein
R₃, Rₐ, R_{b}, X, n and Q are as defined in formula I,
R₁ is selected from Group K with Bz being preferred,
R₂ is selected from Groups E, F and G with Phe being preferred.

The preferred compounds are:

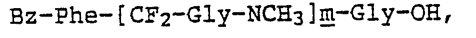

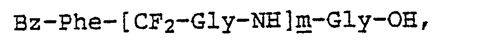

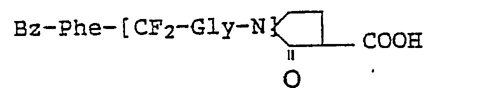

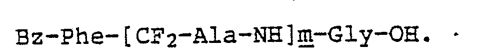

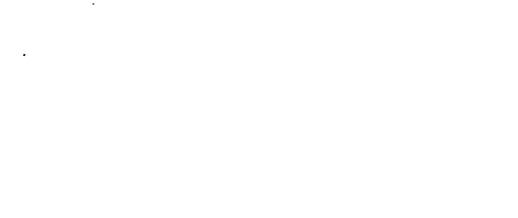

The compounds of formula (Ir) inhibit ACE and are therefore useful as antihypertensives useful in treating hypertension. For their end-use application, the potency and other biochemical parameters of the enzyme inhibiting characteristics of the compounds of (Ir) are readily ascertained by standard biochemical techniques well know in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of enkephalinase are represented by the structural formula 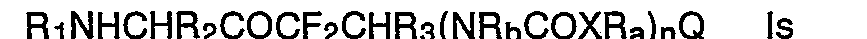 and the hydrates, isosteres or the pharmaceutically acceptable salts thereof, wherein
Rᵢ generically is -P₂P₃, with
P₂ being Gly and
P₃ being selected from Group F or is deleted with Tyr being preferred, and
R₂ is Gly, with
Rs, Rₐ, R_{b}, n, and Q are as defined in I with
Rₐ preferably being methionine or leucine, and
Q preferably is COY with
Y being OH, and
n is preferably one.

The preferred compounds are:

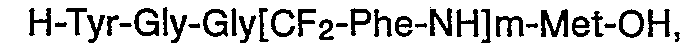

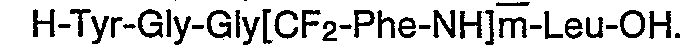

The compounds of formula (Is) inhibit enkephalinase and therefore are useful as analgesics. For their end-use application, the potency and other biochemical parameters of the enzyme inhibiting characteristics of the compounds of (Is) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of pseudomonas elastase are represented by the structural formula 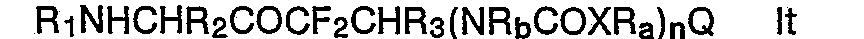 and the hydrates, isosteres or the pharmaceutically acceptable salts thereof, wherein
Rs, Ra, R_{b}, X, n and Q are as defined in formula I,
R₁ is -P₂Ps with
P₂ being selected from Group E with Ala being preferred,
P₃ is selected from Group K with MeOSuc being preferred,
R₂ is selected from Groups E and G with Ala being preferred.

The preferred compound is

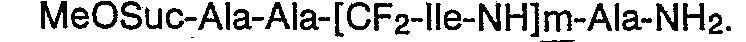

The compounds of formula (It) inhibit pseudomonas elastase and therefore are useful as antibacterial agents particularly useful against infections caused by pseudomonas bacteria. For their end-use application, the potency and other biochemical parameters of the enzyme inhibiting characteristics of the compounds of (It) are readily ascertained by standard biochemical techniques well known in the art. Actual dose ranges for their specific end-use application will, of course, depend upon the nature and severity of the disease state of the patient or animal to be treated as determined by the attending diagnostician. It Is to be expected that the general end-use application dose range will be about 0.01 to 10 mg/kg per day for an effective therapeutic effect with 0.1 mg to 10 mg/kg per day being preferred.

The compounds of formula I which are useful as inhibitors of leucine aminopeptidase are represented by the structural formula 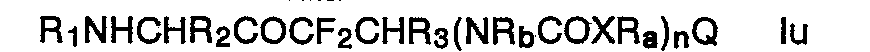 and the hydrates, isosteres or the pharmaceutically acceptable salts thereof, wherein
Rs, Rₐ, R_{b}, X, n and Q are as defined in formula I,
Ri is hydrogen, and
R₂ is selected from Groups A, B, E, F and J with Phe, Leu, Glu and with Arg being preferred.

The preferred compounds are:

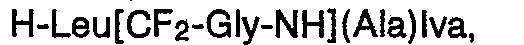

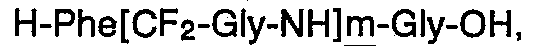

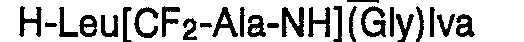

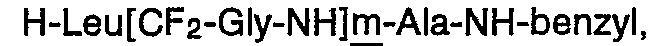

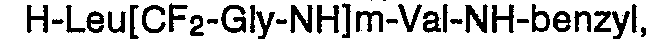

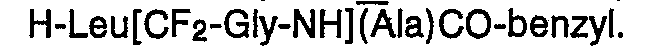

The compounds of formula (lu) are inhibitors of leucine amino peptidase and therefore are useful as immunostimulants useful in conjunctive therapy In the treatment with other known anticancer agents. For their end-use application, the potency and other biochemical parameters of the enzyme inhibiting characteristics of the compounds of (lu) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as Inhibitors of kallikreins, tissue or plasma, are represented by the structural formula 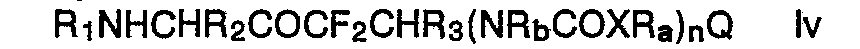 and the hydrates, isosteres or the pharmaceutically acceptable salts thereof, wherein
Rs, Rₐ, R_{b}, X, n and Q are as defined in formula I,
R₁ is -P₂P₃ with
P₂ being selected from Groups E and F with Phe being preferred,
P₃ being selected from Groups C, E or F, the residues of which may be in either the D- or L-configuration, and R₂ preferably is the Arg or J-1 residue.

The preferred compounds of this formula are:

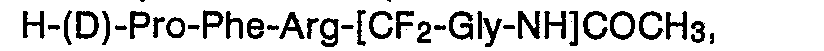

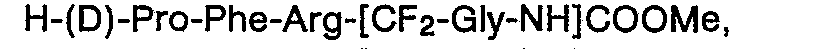

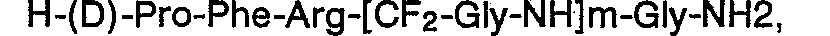

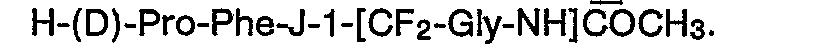

The compounds of the formula (lv) are inhibitors of the kallikreins, tissue or plasma, and therefore inhibit kinin formations. Kinins are generally known to induce pain and vascular permeability associated with inflammation and infection, e.g., bacterial and viral. The inhibition of the kinin formation renders these compounds useful in the alleviation of pain and inflammation. Furthermore, these compounds are useful as male contraceptives in that they will dramatically Interfere with normal sperm function. In their end-use application dose range will be about 0.01 to 10 mg/kg per day for an effective therapeutic effect with 0.1 mg to 10 mg/kg per day being preferred.

From the above, it is obvious that in all of the foregoing instances of (la) through (Iv), the definitions of R₃, R_{b}, Rₐ, X, n and Q as defined in the generic formula I with the specific preferred embodiments being further illustrated for each group of enzyme inhibitors. Of course, it is also understood that in those Instances wherein the carbonyl moiety of Pᵢ is in its reduced form, then such compounds are not hydrates.

Having defined the scope of the compounds within the generic invention and within the individual subgeneric groups for each of the individual enzymes, the manner in which such may be prepared will be described and illustrated.

In general, the compounds of formula I may be prepared using standard chemical reactions analogously known in the art. The key intermediates required for the application of standard peptide coupling techniques may be represented by the formula Va or Vb 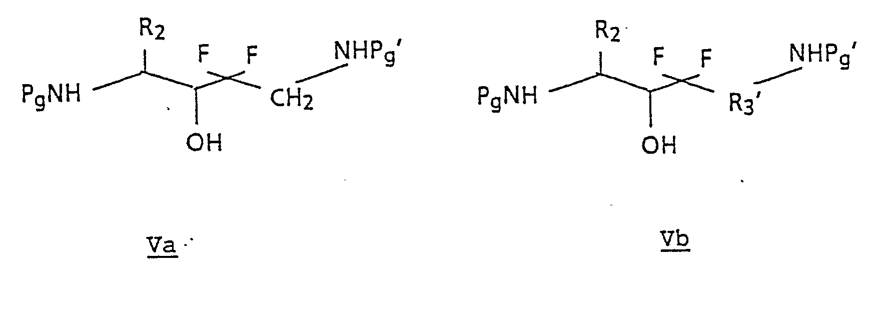 wherein
Rₛ' is as defined for R₃ and may be a protected form of the residue of the specific a-amino acid involved, R₂ is as previously defined, and
Pg and Pg' are each protecting groups, preferably different from each other so as to facilitate selective removal depending upon the site, nature and sequence of the reactions required to prepare the final compounds from these intermediates; the selection being according to principles well known and understood by those in the art.

In those instances wherein Rₛ' represents hydrogen the preparation of the required intermediates (Va) is illustrated by Reaction Scheme A. In those instances wherein Rs' is other than hydrogen then the required intermediates (Vb) are prepared by the methods depicted in Reaction Scheme B.

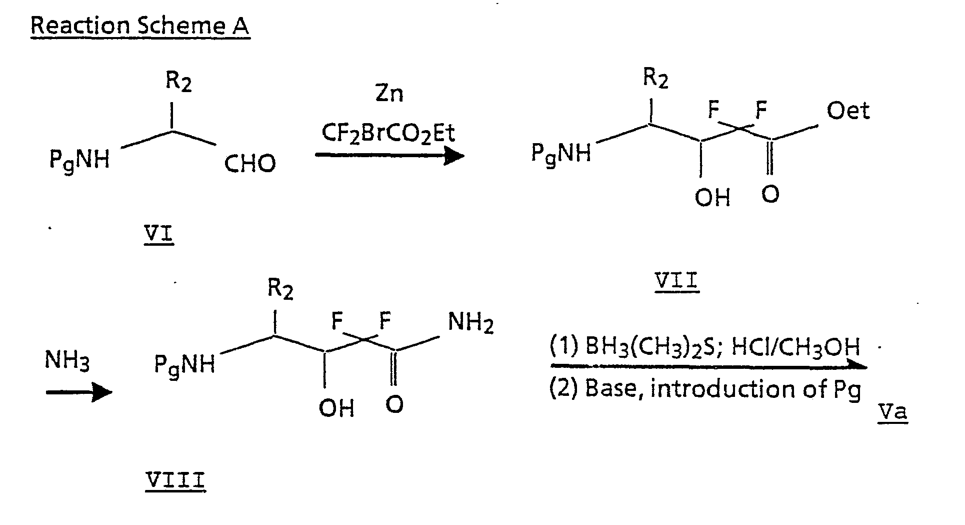

In effecting the steps of Reaction Scheme A it is preferred to start with the aldehyde of formula VI wherein the protecting group is a carbamate preferably wherein Pgis benzyloxycarbonyl (CBZ). This so-protected aldehyde is subjected to a condensation reaction with an ester of bromodifluoroacetic acid, preferably the ethyl ester in the presence of zinc. Preferably the reaction is conducted in an anhydrous aprotic solvent, e.g., tetrahydrofuran, ether, dimethoxyethane and the like under a nitrogen atmosphere. The reaction mixture is gently heated under reflux conditions, preferably to about 60°C for about 1-12 hours. The ester (VII) is converted to its primary amide (VIII) by treatment with liquid ammonia under anhydrous conditions, preferably using such solvents as anhydrous diethyl ether. The amidation is initiated at -78°C and following saturation with ammonia the reaction mixture is slowly allowed to rise to room temperature. The so-formed amide is chemically reduced to form the free amine. This chemical reduction is easily effected by reacting the amide with a diborane, preferably as a diborane/dimelthylsulfide complex, under a nitrogen atmosphere in an anhydrous aprotic solvent (e.g., THF) under reflux conditions. The reduction yields the desired amine, in the form of an acid (e.g., HCI) salt which by pH adjustment yields the free amine which may be suitably protected with an N-protecting group, e.g., Pg' is t-butoxy carbonyl using the standard reaction conditions (e.g., (BOC)₂0, tetrahydrofuran at room temperature) for protecting the amine. Alternatively the free amine may be subjected to reaction conditions designed to build the desired a-amino acid or peptide moiety on the P' side of the difluoromethylene moiety.

In those instances where Rs' is other than hydrogen then the procedure of Reaction Scheme A is modified to prepare the desired intermediates according to Reaction Scheme B. 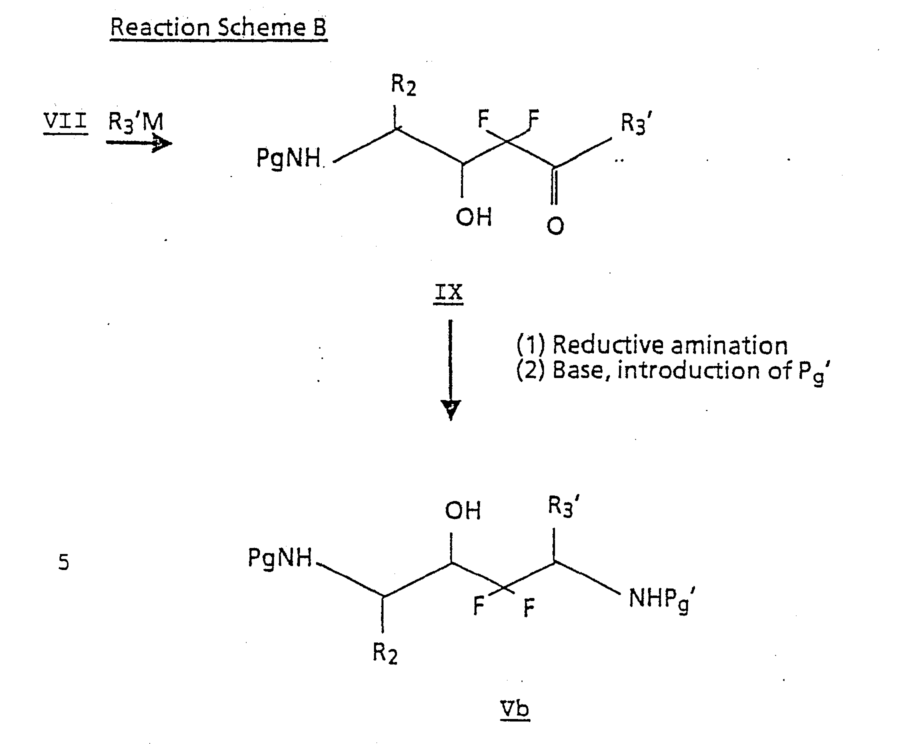 wherein Rs'M is an organometallic reagent, preferably lithium or magnesium coupled to the Rₛ' moiety (other than hydrogen) desired.

The conversion of the ester (VII) to the corresponding Rs' bearing ketone with the organometallic reactant is effected by contacting the reactants together under anhydrous conditions at temperatures of about 0°-80°C in an aprotic solvent (e.g., tetrahydrofuran). Upon reaction the temperature is slowly allowed to rise to room temperature and the complex is hydrolysed to yield the desired intermediate ketones (IX) which compounds are subjected to reductive amination procedures well known in the art, such as, for example, the procedure described by Borch (see R.F. Borch, et al., J. Am. Chem. Soc., 93, 2897 (1971). This reductive amination can take place in one or two steps (with isolation of the intermediate imine or enamine). For example, reacting the ketones (lX) with ammonium acetate under slightly acidic conditions In methanol produces the enamine which, when reacted with sodium cyanoborohydride, produces the desired product. Alternatively, the ketones may be treated directly with sodium cyanoborohydride to produce the desired product. Alternatively, the ketones may be treated directly with sodium cyanoborohydride in the presence of ammonium acetate to produce the desired amines (as its HCI salts) which, in either case, may be neutralized and then the NH₂ moiety may be protected with an appropriate protecting group.

Having obtained the key intermediates of formula V (a and b) standard a-amino acid or peptide coupling procedures may be conducted to prepare the individual compounds of formula I. In practice it is more convenient to effect coupling on the P' side of the difluoromethylene moiety before coupling the P₂-Pn moieties because the CBZ protecting group is generally more stable and this facilitates a less difficult route of synthesis for the desired compounds. In general, this series of reactions may be depicted by Reaction Scheme C.

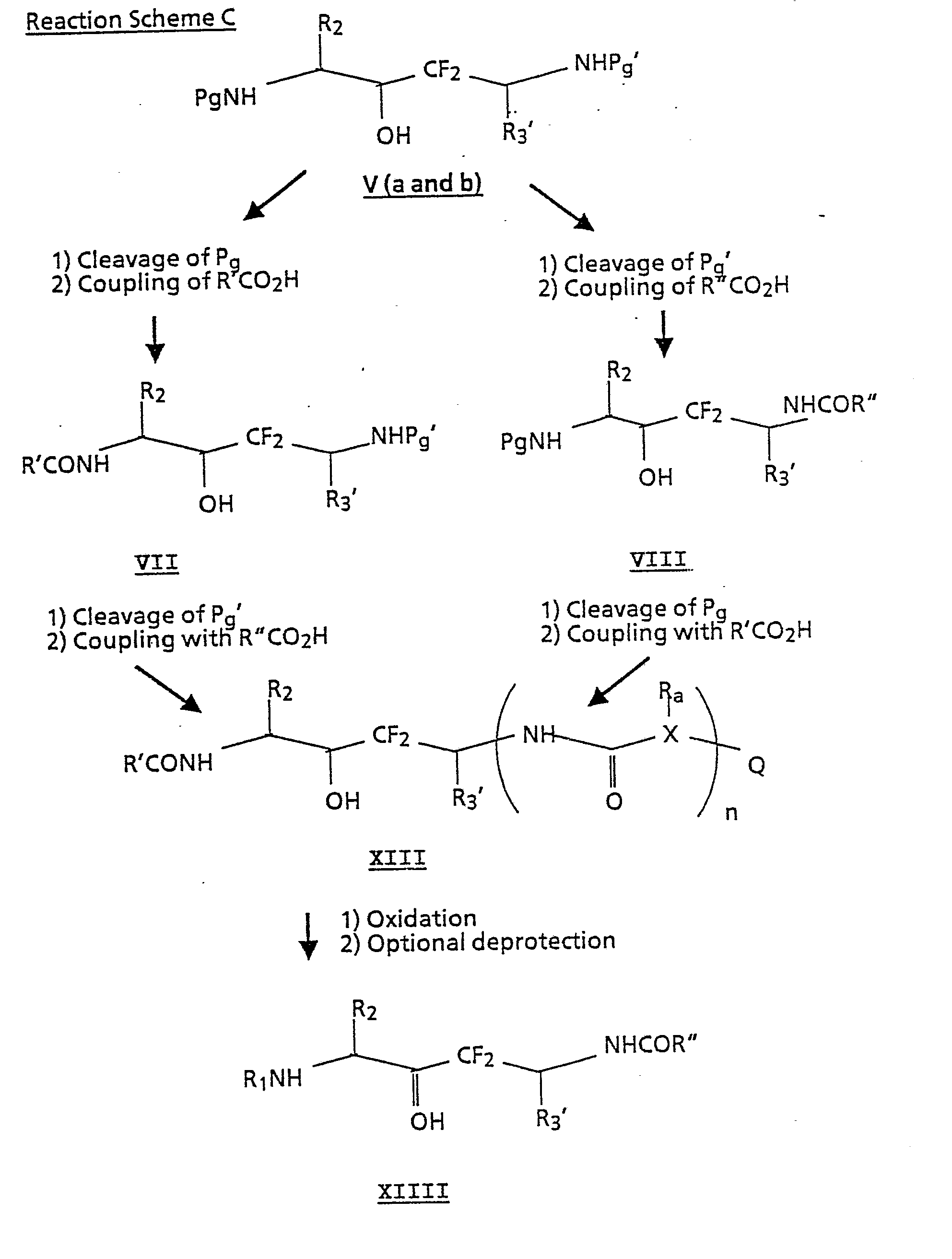

Formula XIII may otherwise be written as 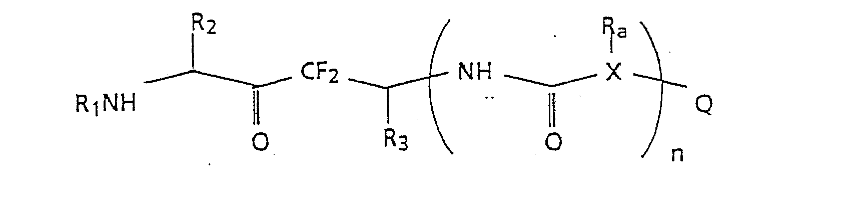 wherein Pg, R₂, R₃, Pg', R₁ are as previously defined and R'C0₂H (leading to VII) is the equivalent of R₁0H of formula I, and R"C0₂H is the equivalent of (NHCOXRq)_{nQ} of formula I, for example, n is one and R"CO is COXRₐQ. The oxidation may be effected via the well-known Swern oxidation procedure, or with a modified Jones reaction using pyridinium dichromate, or a chromic anhydride-pyridinium complex, or with 1,1,1-triacetoxy-2,1-benzoxiodol. Of course, if there are any protecting groups on the residues of the a-amino acid building blocks, such protecting groups may be removed after oxidation. The coupling procedures are effected according to standard procedures well known in the art.

In general the Swern oxidation is effected by reacting about 2 to 10 equivalents of dimethylsulfoxide (DMSO) with about 1 to 6 equivalents of trifluoromethylacetic anhydride [(CF₃CO)₂O] or oxalyl chloride [(COCl)₂], said reactants being dissolved in an inert solvent, e.g., methylene chloride (CH2CI₂), said reactor being under an inert atmosphere (e.g., nitrogen or equivalently functioning gas) under anhydrous conditions at temperatures of about -80⁰ C to -50°C to form an in situ sulfonium adduct to which is added about 1 equivalent of the appropriate alcohols, i.e., from compounds VII and VIII by the coupling with R"C0₂H and R'C0₂H, respectively, having the formula

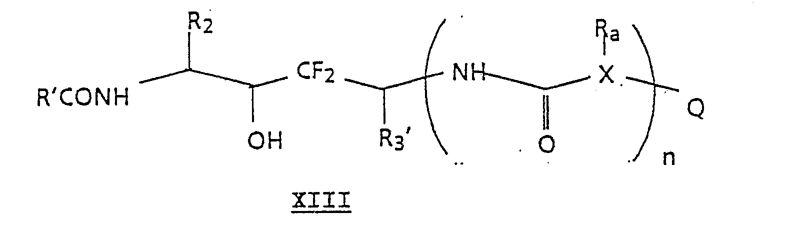

Preferably, the alcohols are dissolved in an inert solvent, e.g., CH₂CI₂ or minimum amounts of DMSO, and the reaction mixture is allowed to warm to about -50°C (for about 10-20 minutes) and then the reaction is completed by adding about 3 to 10 equivalents of a tertiary amine, e.g., triethylamine, N-methyl morpholine, etc.

In general, the modified Jones oxidation procedure may conveniently be effected by reacting the alcohols (XII) with pyridinium dichromate by contacting the reactants together in a water-trapping molecular sieve powder, e.g., a grounded 3 Angstrom molecular sieve), wherein said contact is in the presence of glacial acetic acid at about 0°C to 50°C, preferably at room temperature followed by Isolation and then optionally removing amine protecting groups.

Alternatively, 1 to 5 equivalents of a chromic anhydride-pyridine complex (i.e., a Sarett reagent prepared in situ (see Fieser and Fieser "Reagents for Organic Synthesis" Vol. 1, pp. 145 and Sarett, et al., J.A.C.S. 25, 422, (1953)) said complex being prepared in situ in an inert solvent (e.g., CH₂Cl₂) in an Inert atmosphere under anhydrous conditions at 0° C to 50°C to which complex is added 1 'equivalent of the alcohols (XII) allowing the reactants to interact for about 1 to 15 hours, followed by isolation and optionally removing amine protecting groups.

Another alternative process for converting the alcohols (XII) to the desired ketones (XIII) is an oxidation reaction which employs periodane (i.e., 1,1,1-triacetoxy-2,1-benzoxiodol, (see Dess Martin, J. Org. Chem. 48, 4155, (1983)). This oxidation is effected by contacting about 1 equivalent of the alcohols (XII) with 1 to 5 equivalents of periodane (preferably 1.5 equivalents), said reagent being in suspension in an inert solvent (e.g., methylene chloride) under an inert atmosphere (preferably nitrogen) under anhydrous conditions at 0°C to 50°C (preferably room temperature) and allowing the reactants to interact for about 1 to 48 hours. Optional deprotection of the amine protecting groups may be effected as desired after the ketones have been isolated.

The following specific examples are given to illustrate the preparation of this invention although the scope of compounds is meant to be limiting to the scope of compounds embraced by formula I.

### EXAMPLE 1

### 4-Benzyloxycarbonylamino-2,2-difluor-3-hydroxy-6-methylheptanoic acid, ethyl ester

A mixture of 2.080 g (8.3 mmol) of L-N-benzyloxycarbonyl Leucinal and 2.230 g (11 mol) of ethyl bromodifluoroacetate in dry THF (15 ml) was added dropwise to a refluxing suspension of 0.710 g of activated zinc wool in dry tetrahydrofuran (10 ml), under nitrogen. The addition rate was adjusted to maintain gentle reflux of the mixture. After the addition was complete, the solution was stirred for 3 hours at room temperature. The mixture was quenched by addition of 20 ml ethyl acetate, brine and 1M KHS04 (20 ml). The aqueous layer was dried over anhydrous MgS04, evaporated and purified by flash chromatography (silica gel, ethyl acetate/cyclohexane, 1:9). 1.130 g of the expected ester were isolated (yield:36%) (colorless oil).

Rf: 0.57 (ethyl acetate/cyclohexane, 1:1).

### EXAMPLE 2

### 4-Benzyloxycarbonylamino-2,2-difluoro-3-hydroxy-6-methyl-heptanamide

A stream of dry ammonia was bubbled at -78°C, through a solution of 0.820 g (2.2 mmol) of 4-benzyloxycarbonylamino-2,2-difluoro-3-hydroxy-6-methylheptanoic acid, ethyl ester in anhydrous diethyl ether (10 ml). After saturation, the temperature was allowed to rise to room temperature with stirring. The excess ammonia was removed, and the solvent evaporated in vacuo. The residue was taken off in pentane to yield the expected amide in quantitative yield as a solid.

MS(Cl/NH₃): 345 (MH+).

### EXAMPLE 3

### N⁴-Benzyloxycarbonyl-N¹-tert-butoxycarbonyl-2,2-difluoro3-hydroxy-6-methyl-1,4-heptanediamine

A solution of 1M BH₃/(CH₃)₂S (1 ml) in dichloromethane was added, under nitrogen, to a mixture of 0.185 g (0.53 mmol) of 4-benzyloxycarbonylamino-2,2-difluoro-3-hydroxy-6-methylheptanamide in anhydrous tetrahydrofuran (10 ml). The mixture was heated at reflux for 3 hours. After cooling to room temperature, methanol (3 ml) and 1 N HCI in diethyl ether (6 ml) were added. The solvent was removed in vacuo. The residue was taken off in water and the aqueous layer washed with diethyl ether. The pH of the aqueous phase was adjusted to 10. Diethyl ether extraction afforded the intermediate amine which was directly converted to its N-BOC protected form [(BOC)₂0 1.5 eq; tetrahydrofuran; room temperature]. The expected tert.-butylcarbamate was purified by chromatography (silica gel, ethyl acetate/cyclohexane, 1:1). 0.180 g (79% yield).

Rf: 0.63 (ethyl acetate/cyclohexane, (1:1).

### EXAMPLE 4

### N⁴-Benzyloxycarbonyl-2,2-difluoro-3-hydroxy-6-methyl-1,4-heptanediamine, trifluoroacetate

A solution of 0.320 g (0.75 mmol) of N⁴-benzyloxycarbonyl-N¹-tert-butoxycarbonyl-2,2-difluoro-3-hydroxy-6-methyl-1,3-heptanediamine in trifluoroacetic acid (5 ml) was stirred at 0°C for 30 minutes. The solvent was then removed in vacuo, and the residual oil taken off several times in diethyl ether and evaporated to dryness. The expected amine was obtained in quantitative yield and used in the next step without further purification. The pure free amine was isolated through the following procedure: washing the ethereal solution of the trifluoroacetate salt with saturated sodium bicarbonate (three times). The organic phase was dried over anhydrous magnesium sulphate. Filtration and removal of the solvent in vacuo left the expected pure N⁴⁻benzyloxycarbonyl-2,2-difluoro-3-hydroxy-6-methyl-1,4-heptanediamine as a white solid. (78% yield). Analysis calculated for C₁₆H₂₄N₂0₃F₂

CO/o: 58.17; H%: 7.32; N%: 8.48

found CO/o: 57.66; HO/o: 7.18; N%: 8.31

### EXAMPLE 5

### N⁴-Benzyloxycarbonyl-2,2-difluoro-3-hydroxy-N¹-(2-isovalerylamino-propionyl)-6-methyl-1,4-heptanediamine

To a stirred solution of 0.130 g (0.75 mmol) of N-isovaleryl-D-alanine in dry acetonitrile (5 ml), under nitrogen, was added 0.075 g (0.75 mmol) of N-methyl-morpholine. The resultant solution was cooled to -20° C. Isobutyl chloroformate (0.103 g: 0.75 mmol) was added dropwise to the cooled reaction mixture. After 10 minutes, a mixture of 0.333 g (0.75 mmol) of N4-benzyloxycarbonyl-2,2-difluoro-3-hydroxy-6-methyl-1,4-heptanediamine, trifluoroacetate and 0.080 g'of N-methylmorpholine in dry dimethylformamide (5 ml) was added to the cooled mixture. After stirring for 4 hours at -20°C, the temperature of the mixture was allowed to rise to room temperature. Stirring was continued for 15 hours at room temperature. The mixture was then concentrated and placed under high vacuum to remove all the dimethylformamide. The resultant residue was chromatographed (silica gel, ethyl acetate) to give the expected peptide in 65% yield.

Rf: 0.13 (ethyl acetate/cyclohexane, 1:1).

Analysis calculated for C₂₄H₃₇N₃0₅F₂

C%: 59.36; HO/o: 7.68; N%: 8.65

found CO/o: 59.72; HO/o: 7.72; N%: 8.54.

### EXAMPLE 6

### 2,2-Difluoro-3-hydroxy-N¹-(2-isovalerylaminopropionyl)-6-methyl-1,4-heptanediamine

A solution of 0.192 g (0.39 mmol) of N⁴-benzyloxycarbonyl-2,2-difluoro-hydroxy-N¹-2-(isovalerylaminopro- pionyl)-6-methyl-1,3-heptanediamine in ethanol (20 mt) was stirred at room temperature, in the presence of 10⁰/₀ Palladium on charcoal (0.010 g) under a hydrogen atmosphere for 5 hours. The hydrogen atmosphere was then replaced by a nitrogen atmosphere and the catalyst was filtered. The solvent was removed in vacuo leaving 0.125 g of a white solid (82% yield).

### EXAMPLE 7

### 2,2-Difluoro-3-hydroxy-N⁴-(2-isovalerylaminoisovaleryl)-N¹-(2-isovalerylaminopropionyl)-6-methyl-1,3-hep- tanediamine

The title compound was obtained from the amine of Example 6 and N-isovaleryl-L-valine by the procedure described in Example 5.

Rf: 0.45 (methanol/chloroform, 8:92).

MS(Cl/NH₃): 535 (MH+).

### EXAMPLE 8

### 2,2-Difluoro-N⁴-(2-isovalerylaminoisovaleryl)-N¹-(2-isovalerylaminopropionyl)-6-methyl-3-oxo-1,4-heptane- diamine

A solution of 0.024 g (0.045 mmol) of 2,2-difluoro-3-hydroxy-N⁴-(2-isovalerylaminoisovaferyl)-N¹-(2-isovale- rylaminopropionyl)-6-methyl-1,4-heptanediamine in methylene chloride (5 ml) was added to a suspension of pyridinium dichromate (0.026 g) and 3A molecular sieves (0.038 g), containing 4 liters of glacial acetic acid. Stirring was continued for 15 hours at room temperature. Florisil (0.080 g) was added, stirring continued for 15 minutes and the mixture filtered over sand. Removal of the solvent and chromatography (silica gel, ethyl acetate/acetone, 7:3) afforded the expected difluoroketone as a white solid (0.013 g; 55% yield).

Rf: 0.46 (methanol/chloroform 8:92)

MS(CI/NHs): 532 (M+).

### EXAMPLE 9

N⁴-Benzyloxycarbonyl-2,2-difluoro-3-hydroxy-N¹-(2-isopentylaminocarbonylpropionyl)-6-methyl-1,4-heptane-

### diamine

To a solution of 0.155 g (0.82 mmol) of 2-isopentylaminocarbonylpropanoic acid, 0.126 g of 1-hydroxybenzotriazole-H₂0 and 0.169 g of N,N'-dicyclohexylcarbodiimide in anhydrous methylene chloride (5 ml), at 0°C was added a mixture of 0.363 g (0.82 mmol) of N⁴⁻benzyloxycarbonyl- 2,2-difluoro-3-hydroxy-6-methyl-1,4-heptanediamine, trifluoroacetate and 0.083 g (0.82 mmol) of N-methylmorpholine in methylene chloride (3 ml). The cooling bath was removed after 1 hour and the reaction was stirred at room temperature overnight. The reaction mixture was then filtered and the filtrate was concentrated in vacuo. The expected peptide was isolated in 83% yield (0.340 g) after column chromatography purification (silica gel, ethyl acetate/chloroform, 1:1).

Rf: 0.16 (ethyl acetate/chloroform, 1:1).

Analysis calculated for C₂₆H₃₉N₃O₅F₂

C%: 60.10; HO/o: 7.87; NO/o: 8.41

found C%: 60.26; H%: 7.81; N%: 8.33.

### EXAMPLE 10)

### 2,2-Difluoro-3-hydroxy-N¹-(2-isopentyfaminocarbonytpropionyl)-6-methyl-1,4-heptanediamine

The title compound was prepared from the peptide of Example 9 by the procedure described in Example 6 (96% yield).

### EXAMPLE 11

### 2,2-Difluoro-3-hydroxy-N⁴-(2-isovalerylaminoisovalery)-N¹-(2-isopentylaminocarbonylpropionyl)-6-methyl-1,4-heptanediamine

The title compound was prepared from the amine of Example 10 and N-isovaleryl-L-valine by the procedure described in Example 9.

Rf: 0.41 (methanol/chloroform, 8:92)

MS(CI/NH₃): 549 (MH+).

### EXAMPLE 12

### 2,2-Difluoro-N4-(2-isovalerylaminoisovaleryl)-N1-(2-isopentylaminocarbonylpropionyl)-6-methyi-3-oxo-1,4-heptanediamine

The title compound was prepared from the alcohol of Example 11 by the procedure described in Example 8 (75% yield).

MS(Cl/NH₃): 547 (MH⁺).

### EXAMPLE 13

### 4-Benzyloxycarbonylamino-2,2-difluoro-3-hydroxy-5-phenylpentanoic acid, ethyl ester

The title compound was prepared from L-N-benzyloxycarbonylphenylalaninal and ethyl bromodifluoroacetate by the procedure described in Example 1 (75% yield).

Rf: 0.5 (ethyl acetate/cyclohexane, 1:1).

Analysis calculated for C₂₁H₂₃NO₅F₂

C%: 61.91; H%: 5.69; N%: 3.44

found C%: 62.19; H^{O}/o: 5.75; N%: 3.55 .

### EXAMPLE 14

### 4-Benzyloxycarbonylamino-2,2-difluoro-3-hydroxy-5-phenylpentanamide

The title compound was prepared from the ester of Example 13 by the procedure described in Example 2 (98% yield).

### EXAMPLE 15

### N⁴-Benzyloxycarbonyl-N¹-tert-butoxycarbonyl-2,2-difluoro-3-hydroxy-5-phenyl-1,4-pentanediamine

The title compound was prepared from the amide of Example 14 by the procedure described in Example 3 (64% yield).

### EXAMPLE 16

### N¹-tert-Butoxycarbonyl-2,2-difluoro-3-hydroxy-5-phenyl-1,4-pentanediamine

The title compound was prepared in quantitative yield from the dicarbamate of Example 15 by the procedure described in Example 6.

### EXAMPLE 17

### N⁴-Benzoyl-N¹-tert-butoxycarbonyl-2,2-difluoro-3-hydroxy-5-phenyl-1,4-pentanediamine

A solution of 0.330 g (1.03 mmol) of N¹-tert-butoxycarbonyl-2,2-diffuoro-3-hydroxy-5-phenyl-1,4-pentanediamine and 0.145 g benzoyl chloride (1.03 mmol) in anhydroud tetrahydrofuran was stirred at room temperature for 14 hours in the presence of 0.101 g triethylamine (1 mmol). The solvent was removed in vacuo. The residue was taken off in a mixture of methylene chloride and water. The organic phase was dried over MgS0₄. Evaporation and recrystallization from ethyl acetate/pentane afforded the expected compound as a white solid.

Analysis calculated for C₂₃H₂₈Bz0₄F₂

C%: 63.58; H%: 6.49; N%: 6.45

found C%: 63.68; HO/o: 5.75; N%: 6.85

### EXAMPLE 18

### N⁴-Benzoyl-2,2-difluoro-3-hydroxy-5-phenyl-1,4-pentanediamine, trifluoroacetate

The title compound was prepared in quantitative yield from the product of Example 17 by the procedure of Example 4.

### EXAMPLE 19

### N¹-Acetyl-N4-benzoyl-2,2-difluoro-3-hydroxy-5-phenyl-1,4-pentanediamine

The title compound was prepared from the amine of Example 18 and acetic anhydride by the procedure described in Example 17 in the presence of 2 equivalents of N-methyl-morpholine.

MS(CI/NHs): 377 (MH+).

### EXAMPLE 20

### N ¹-Acetyl-N4-benzoyl-2,2-diffuoro-oxo-5-phenyl-1,4-pentanedlamine

The title compound was prepared from the alcohol of Example 19 by the procedure described in Example 8. Rf: 0.25 (methanol/chloroform, 8:92).

MS(Cl/NH₃): 375 (MH+).

### EXAMPLE 21

### 4-Benzyloxycarbonylamino-2,2-difluoro-3-hydroxy-5-methyl-hexanolc acid, ethyl ester

The title compound was prepared from L-N-benzyloxycarbonylvalinal and ethyl bromfdifiuoroacetate by the procedure described in Example 1. (40% yield)

### EXAMPLE 22

### 4-Benzyloxycarbonylamino-2,2-difluoro-3-hydroxy-5-methyl-hexanamide

The title compound was prepared in quantitative yield from the ester of Example 21 by the procedure described in Example 2.

### EXAMPLE 23

### N⁴-Benzyloxycarbonyl-N¹-tert-butoxycarbonyl-2,2-difiluoro-hydroxy-5-methyl-1,4-hexanediamine

The title compound was prepared from the amide of Example 22 by the procedure described in Example 3 (yield: 400/o).

Rf: 0.50 (ethyl acetate/cyclohexane, 1:1).

### EXAMPLE 24

### N^{l-}tert-Butoxycarbonyl-2,2-difluoro-3-hydroxy-5-methyl-1,4-hexanediamine

The title compound was prepared in quantitative yield from the dicarbamate of Example 23 by the procedure described in Example 6.

### EXAMPLE 25

### N¹-tert-Butoxycarbonyl-2,2-difluoro-3-hydroxy-N⁴-(methoxysuccinyl-L-alanyl-L-alanyl-L-pralyl)-5-methyl-1,4-hexanediamine

The title compound was prepared from the amide of Example 24 and methoxy succinyl-L-alanyl-L-alanyl-L-proline by the procedure described in Example 5 (yield: 480%).

### EXAMPLE 26

### 2,2-Difluoro-3-hydroxy-N⁴-[methoxysuccinyl-L-alanyl-L-alanyl-L-prolyl]-5-methyl-1,4-hexanediamine, trifluoroacetate

The title compound was prepared in quantitative yield from the product of Example 25 by the procedure described in Example 4.

### EXAMPLE 27

### 2,2-Difluoro-3-hydroxy-N⁴-[methoxysuccinyl-L-alanyl-L-alanyl-L-prolyl]-5-methyl-N¹-(2-methylmalona- moyl)-1,4-hexanediamine

The title compound was prepared from the amine saltof Example 26 and 2-methylmalonamic acid by the procedure described in Example 9.

### EXAMPLE 28

### 2,2-Difluoro-N⁴-[methoxysuccinyl-L-alanyl-L-alanyl-L-prolyl]-5-methyl-N1-(2-methylmalonamoyl)-3-oxo-1,4-hexanediamine

The title compound was prepared from the alcohol of Example 27 by the procedure described in Example 8.

### EXAMPLE 29

### 5-Benzyloxycarbonylamino-3,3-difiuoro-4-hydroxy-7-methyl-2-octanone

A 1.6 M solution of methyllithium in 1 ml of diethyl ether was added at -78°C to a solution of 0.195 g 4-benzyloxycarbanylamino-2,2-dfftuoro-3-hydroxy-6-methylheptanoic acid, ethyl ester (0.5 mmol) in 5 ml dry tetrahydrofuran. After stirring for 1 hour at ₋78°C, the temperature was allowed to rise to room temperature. Stirring was continued for 3 hours at room temperature. The mixture was hydrolyzed and extracted with diethyl ether. The organic layer was washed with brine and dried over MgS0₄. Filtation and removal of the solvent, in vacuo, left an oil, purified by column chromatography (silica gel, ethyl acetate/cyclohexane, 2:8). 0.080 g of the expected ketone was thus isolated as a colorless oil (yield 47%).

Rf: 0.56 (ethyl acetate/cyclohexane, 1:1).

### EXAMPLE 30

### N5-Benzyloxycarbonyl-N2-tert-butoxycarbonyl-3,3-difluoro-4-hydroxy-7-methyl-2,5-octanediamine

A mixture of 0.080 g 5-benzyloxycarbonylamino-3,3-difluoro-4-hydroxy-7-methyl-2-octanone (0.23 mmol),0.177 g ammonium acetate (2.3 mmol), and 0.010 g sodium cyanoborohydride (0.16 mmol) in 3 ml methanol was stirred at room temperature, under nitrogen, for 20 hours. The mixture was acidified by addition of 1 N HCI (2 ml) and the solvent removed in vacuo. The residue was taken off in water. the aqueous phase was washed with diethyl ether. The pH of the aqueous phase was adjusted to 10. Diethyl ether afforded the intermediate amine which was directly converted to its N-BOC protected form. [(BOC)20, 1.5 equivalent; tetrahydrofuran; room temperature]. The expected dicarbamate was purified by chromatography (silica gel, ethyl acetate/cyclohexane, 1:1).

### EXAMPLE 31

### N^{l-}(2-Benzylaminocarbonyl-3-methylbutanoyl)-N⁴⁻(benzyloxycarbonyl)-2,2-difluoro-3-hydroxy-6-methyl-1,4-heptanediamine

The title compound was prepared from the amine of Example 4 and 2-benzylaminocarbonyl-3-methylbutanoic acid by the procedure described in Example 9 (yield: 55%)
Rf: 0.60 (ethyl acetate)
MS(Cl/NH₃): 548 (MH+).
Analysis calculated for C₂₉H₃₉N₃0₅F₂
C%: 63.60; H%: 7.18; N%: 7.67
found C%: 63.71; H%: 7.10; N%: 7.44.

### EXAMPLE 32

### N¹-(2-Benzylaminocarbonyl-3-methylbutanoyl)-2,2-difluoro-3-hydroxy-6-methyl-1,4-heptanediamine

The title compound was prepared from the compound of Example 31 by the procedure described in Example 6 (yield: 970/o).

### EXAMPLE 33

### N¹-(2-Benzylaminocarbonyl-3-methylbutanoyl)-N⁴-[N-benzyloxycarbonyl-Nim-tert-butoxycarbonyl-L-Histidyl]2,2-difluoro-3-hydroxy-6-methyl-1,4-heptanediamine

The title compound was prepared from N-benzyloxycarbonyl-Nim-tert-butoxycarbonyl-L-Histidine and the amine of Example 32 by the procedure described in Example 5 (yield:64%).

Rf: 0.35 (ethyl acetate).

MS: (Cl/NH₃): 785 (MH+).

### EXAMPLE 34

### Nl-(2-Benzylaminocarbonyl-3-methylbtanoyl)-N4-Nim-tert-butoxycarbonyl-L-histidyl)-2,2-difluoro-3-hydroxy-6-methyl-1,4-heptanediamine

The title compound was prepared from the compound of Example 33 by the procedure described in Example 6 (yield: 90%).

### EXAMPLE 35

### N¹⁻(2-Benzylaminocarbonyl-3-methylbutanoyl)-N⁴⁻[[N-benzyloxycarbonyl-3-(1-naphthyl)alanyl]-Nim-tert-butoxycarbonyl-L-histidyl]-2,2-difluoro-3-hydroxy-6-methyl-1,4-heptanediamine

The title compound was prepared from N-benzyloxycarbonyl-3-(1-naphthyl)alanine and the amine of Example 34 by the procedure described in Example 5 (yield: 77%).

### EXAMPLE 36

### N¹-(2-Benzylaminocarbonyl-3-methylbutanoyl)-N⁴-[N-(benzyloxycarbonyl)-L-Phenylalanyl-Nim-tert-butoxycarbonyl-L-histidyl]-2,2-difluoro-3-hydroxy-6-methyl-1,4-heptanediamine

The title compound was prepared from N-benzyloxycarbonyl-L-phenylalanine and the amine of Example 34 by the procedure described in Example 5 (yield: 60%).

### EXAMPLE 37

### N1-(2-Benrylaminocarbonyl-3-methylbutanoyl)-N4-[N-(tert-butoxycarbonyl)-L-phenylalanyl-L-n-valyl}-2,2-di- fluoro-3-hydroxy-6-methyl-1,4-heptanediamine

The title compound was prepared from N-(tert-butoxycarbonyl)-L-phenylalanyl-L-n-valine and the amine of Example 32 by the procedure described in Example 5 (yield: 580/o).

Rf: 0.56 (methanol/chloroform 8:92).

MS(CI/NH₃): 760 (MH+).

### EXAMPLE 38

### N¹-(2-Benzylaminocarbonyl-3-methylbutanoyl)-N⁴-[N-(tert-butoxycarbonyl)-L-phenyfalarₗyl-L-n-valyl]-2,2-di- fluoro-6-methyl-3-oxo-1,4-heptanediamine

The title compound was prepared in 70% yield from the alcohol of Example 37 by the procedure described in Example 8.

Rf: 0.62 (methanol/chloroform 8:92)

MS(CI/NH₃): 758(MH+).

### EXAMPLE 39

### N¹-(2-Benzylaminocarbonyl-3-methylbutanoyl)-N⁴-[N-(benzyoxycarbonyl)-L-phenylalanyl-Nim-(tert-butoxycarbonyl)-L-histidyl]-2,2-difluoro-6-rriethyl-3-oxo-1,4-Heptanediamine

The title compound was prepared in 63% yield from the alcohol of Example 36 by the procedure described in Example 8.

Rf: 0.29 (ethyl acetate)

MS(CI/NH₃): 930(MH+).

### EXAMPLE 40

### N¹-(2-Benzylaminocarbonyl-3-methylbutanoyl)-N⁴-[N-(benzyloxy-carbonyl)-L-phenylalanyl-L-histidyl]-2,2-di- fluoro-6-methyl-3-oxo-1,4-heptanediamine

A mixture of 0.035 g (0.04 mmol) qf N^{l-}(2-benzylaminocarbonyl-3-methylbutanoyl)-N⁴⁻[N-(benzyloxycarbonyl)-L-phenylalanyl-Nim-(tert-butoxycarbonyl)-L-histidyl]-2,2-difluoro-6-methyl-3-oxo-1,4-heptanediamine and trifluoro acetic acid (5 mL) was stirred at 0° C for one hour. The solvent was removed in vacuo. The residue was taken off in ethyl acetate. The organic solution was washed with 50/o sodium bicarbonate and dried over anhydrous magnesium sulphate. Filtration, removal of the solvent in vacuo and purification by flash chromatography (silica gel; chloroform/methanol 98:2 to 92:8) yielded 0.019 g of the title compound as a white solid (61% yield).

MS(DCl/Cl+/NH₃); 830(MH+,₉₀); 722(83); 683(100); 649(21); 575(70); 504(76).

MS(DCl/Cl+/NH₃); 830(MH+,90); 722(83); 683(100); 649(21); 575(70); 504(76).

### EXAMPLE 41

### N⁴-Benzyloxycarbonyl-2,2-difluoro-3-hydroxy-6-methyl-N¹-(3-methyl-2R-phenylacetylamlnobuta- noyl)-1,4-heptanediamine

The title compound was prepared in 38% yield from the amine of Example 4 and N-phenylacetyl-D-valine by the procedure described in Example 9.

Rf: 0.52 (silica gel; ethyl acetate)

MS: 565(MNH⁺, 57); 548(MH+, 38); 457(85); 440(63); 414(100); 324(11).

### EXAMPLE 42

### 2,2-Difluoro-3-hydroxy-6-methyl-N¹-(3-methyl-2R-phenylacetyfaminobutanoyl)-1,4-heptanediamine

The title compound was prepared in 63% yield from the carbamate of Example 41 by the procedure described in Example 6.

### EXAMPLE 43

### N⁴-[N-(tert-butoxycarbonyl)-L-phenylalanyl-L-n-valyl]-2,2-difiporo-3-hydroxy-6-methyl-N¹-(3-methyl-2R-phe- nylacetylaminobutanoyl)-1,4-heptanediamine

The title compound was prepared in 97% yield from N-(tert-putoxycarbonyl)-L-phenylalanyl-L-n-valine and the amine of Example 42 by the procedure described in Example 9 (solvent used: methylene chloride/dimethylformamide 3:1).

MS(DCl/Cl+/NH₃); 777(MNH₄⁺, 66); 760(MH₄⁺, 100); 703(20), 686(15), 660(15).

### EXAMPLE 44

### N⁴-[N-(tert-butoxycarbonyl)-L-phenylalanyl-L-n-valyl]-2,2-difluoro-6-methyl-N¹-(3-methyl-2R-phenylacety- laminobutanoyl)-3-oxo-1,4-heptanediamine

The title compound was prepared in 50% yield from the alcohol of Example 43 by the procedure described in Example 8.

MS: 775(MNH⁺₄, 30), 758MH+, 100).

### EXAMPLE 45

### N¹-(2-Benzylaminocarbonyl-3-methylbutanoyl)-N⁴-[N-(benzyloxycarbonyl)-L-phenylalanyl-L-n-valyl]-2,2-diflu- oro-3-hydroxy-6-methyl-1,4-heptanediamine

The title compound was prepared in 77% yield from N-(benzyloxycarbonyl)-L-phenylalanyl-L-n-valyl and the amine of Example 32 by the procedure described in Example 9.

Rf: 0.47 (silica gel; chloroform/methanol 92:8)

MS(DCI/CI+/NH₃): 811(MNH⁺₄, 25); 794(MH+, 9); 703(100); 686(18); 660(5); 613(7).

### EXAMPLE 46

### N¹⁻(2-Benzylaminocarbonyl-3-methylbutanoyl)-N⁴⁻[N-(benzyloxycarbonyl)-L-phenylalanyl-L-n-valyl]-2,2-diflu- oro-6-methyl-3-oxo-1,4-heptanediamine

The title compound was prepared in 82% yield from the alcohol of Example 45 by the procedure described in Example 8.

Rf: 0.64 (silica gel; chloroform/methanol 92:8)

MS(DCI/Cl+/CH₄): 792(MH+) >

### EXAMPLE 47

### N¹-(2-Benzylaminocarbonyl-3-methylbutanoyl)-N⁴-[N-(benzyloxycarbonyl-Nim-tert-butoxycarbonyl-L-histidyl]-2,2-difluoro-6-methyl-3-oxo-1,4-heptanediamine

The title compound was prepared in 60% yield from the alcohol of Example 33 by the procedure described in Example 8.

Rf: 0.44 (silica gel; ethyl acetate)

MS(DCI/CI+/NH₃): 783(MH+, 40); 683(66); 575(38); 457(100).

### EXAMPLE 48

### N¹-(2-Benzylaminocarbonyl-3-methylbutanoyl)-N⁴-[N-(benzyloxycarbonyl-L-histidyl]-2,2-diffuoro-6-methyl-3-oxo-1,4-heptanediamine

The title compound ws prepared in 54% yield from the ketone of Example 47 by the procedure described in Example 40.

Rf: 0.19 (silica gel; chloroform/methanol 92:8)

MS(DCI/CI+/NH₃): 683(MH+, 100); 575(60).

### EXAMPLE 49

### N¹⁻(2-Benzylaminocarbonyl-3-methylbutanoyl)-N⁴⁻[[N-(benzyloxycarbonyl-3-(1-naphthyl)alanyl]-Nim-ₜert-butoxycarbonyl-L-histidyl]-2,2-difluoro-6-methyl-3-oxo-1,4-heptanediamine

The title compound was prepared in 50% yield from the alcohol of Example 35 by the procedure described in Example 8.

Rf: 0.36 (silica gel; ethyl acetate)

MS(DCI/CI+/NH₃): 980(MH+, 2); 880(16); 772(100); 753(26); 732(90); 575(23); 498(100).

### EXAMPLE 50

### N¹⁻(2-Benzylaminocarbonyl-3-methylbutanoyl)-N⁴⁻[[N-(benzyloxycarbonyl-3-(1-naphthyl)alanyl]-L-histidyl]-2,2-difluoro-6-methyl-3-oxo-1,4-heptanediamine

The title compound was prepared from the carbamate of Example 49 by the procedure described in Example 40.

### EXAMPLE 51

### N¹-(2-Benzylaminocarbonyl-3-methylbutanoyl)-N⁴-[N-(tert-butoxycarbonyl)-L-phenylalanyl-N^{Im}-(tert-butoxycarbonyl)-L-histidyl]-2,2-difluoro-3-hydroxy-6-methyl-1,4-heptanediamine

The title compound was prepared in 500/o yield from (N-tert-butoxycarbonyl)-L-phenylalanine and the amine of Example 34 by the procedure described in Example 5.

Rf: 0.43 (silica gel; ethyl acetate) .

### EXAMPLE 52

### N¹-(2-Benzylaminocarbonyl-3-methylbutanoyl)-N⁴-[N-(tert-butoxycarbonyl)-L-phenylalanyl-Nim-(tert-butoxycarbonyl)-L-histidyl]-2,2-difluoro-6-methyl-3-oxo-1,4-heptanediamine

The title compound was prepared in 530/o yield from the alcohol of Example 51 by the procedure described in Example 8.

Rf: 0.57 (silica gel; ethyl acetate)

MS(DCl/Cl+/NH₃): 896.5 (MH+, 24); 796.4(76); 696.4(4); 649.4(100).

### EXAMPLE 53

### N¹-(2-Benzylaminocarbonyl-3-methylbutanoyl)-N⁴-[N-(tert-butoxycarbonyl)-L-phenylalanyl-L-histidyl]-2,2-di- fluoro-6-methyl-3-oxo-1,4-heptanediamine

To a solution of N¹-(2-benzylaminocarbonyl-3-methylbutanoyl)-N⁴-[N-(tert-butoxycarbonyl)-L-phenylalanyl-N^{im}-(tertbutoxycarbonyl)-L-histidyl]-2,2-difluoro-6-methyl-3-oxo-1,4-heptanediamlne (0.179 g, 0.2 mmol) in methanol (3 mL) was added anhydrous potassium carbonate (0.063 g, 0.46 mmof). The mixture was stirred 1.5 hours at room temperature. Acetic acid (0.2 mL) was added and the mixture was evaporated to dryness, at reduced pressure. The residue was taken off in ethyl acetate. The organic solution was washed with 5% sodium bicarbonate, and dried over anhydrous magnesium sulphate. Filtration, removal of the solvent in vacuo and purification by flash chromatography yielded the title compound as a white solid.

### EXAMPLE 54

### N¹-(2-Benzylaminocarbonylpropanoyl)-N⁴-(benzyloxycarbonyl)-2,2-difluoro-3-hydroxy-6-methyl-1,4-heptane- diamine

The title compound was prepared in 81% yield from 2-benzylaminocarbonylpropanoic acid and the amine of Example 4 by the procedure described in Example 9.

Rf: 0.48 (silica gel; ethyl acetate)

MS(DCI/CI+/NH₃): 537((MNH⁺₄, 96); 520(MH+, 100); 420(10); 386(28); 242(37); 225(89).

### EXAMPLE 55

### N¹-(2-Benzylaminocarbonylpropanoyl)-2,2-difluoro-3-hydroxy-6-methyl-1,4-heptanediamine

The title compound was prepared in 87% yield from the carbamate of Example 54 by the procedure described in Example 6.

### EXAMPLE 56

### N¹-(2-Benzylaminocarbonylpropanoyl)-N⁴-[N-(tert-butoxycarbonyl)-L-phenylalanyl-L-n-valyl]-2,2-difluoro-3-hydroxy-6-methyl-1,4-heptanediamine

The title compound was prepared in 66% yield from N-(tert-butoxycarbonyl)-L-phenylalanyl-L-n-valine and the amine of Example 55 by the procedure described in Example 5.

Rf: 0.40 (silica gel; chloroform/methanol 92:8)

MS(DCI/CI+/NH₃: 749 (MNH⁺₄, 28); 732(MH+, 82); 632(39); 282(96); 265(100).

Analysis calculated for C₃₈H₅₅N₅O₇F₂: C%: 62.36; H%: 7.57; N%: 9.57. Found: C%: 62.13; H%: 7.59; NO/o: 9.34.

### EXAMPLE 57

### N¹-(2-Benzylaminocarbonylpropanoyl)-N⁴-[N-(tert-butoxycarbonyl-L-phenylalanyl-L-n-valyl]-2,2-difluoro-6-methyl-3-oxo-1,4-heptanediamine

The title compound was prepared in 790/o yield from the alcohol of Example 56 by the procedure described in Example 8.

Rf: 0.52 (silica gel; chloroform/methanol 92:8)

MS(DCl/Cl+NH₃): 748(MNH⁺₄, 16); 731(MH+, 100).

Analysis calculated for : C₃₈H₅₃N₅O₇F₂; C%: 62.53; HO/o: 7.32; NO/o: 9.59. Found C%: 62.10; HO/o: 7.37; N%: 9.53.

### EXAMPLE 58

### N¹-(2-Benzylaminocarbonylacetyl)-N⁴-(benzyloxycarbonyf)-2,2-difluoro-3-hydroxy-6-methyl-1,4-heptane- diamine

The title compound was prepared in 65% yield from 2-benzylaminocarbonylacetic acid and the amine of Example 4 by the procedure described in Example 9.

Rf: 0.32 (silica gel; ethyl acetate)

MS(DCl/Cl+/NH₃): 523(MNH⁺₄, 100); 506(MH+, 26).

Analysis calculated for C₂₆H₃₃N₃0₅F₂: C%: 61.77; H%: 6.58; N%: 8.31. Found: C%: 61.50; H%: 6.59; NO/o: 8.23.

### EXAMPLE 59

### N¹-(2-Benzylaminocarbonylacetyt)-2,2-difluoro-3-hydroxy-6-methyl-1,4-heptanediamine

The title compound was prepared in 93% yield from the carbamate of Example 58 by the procedure described in Example 6.

### EXAMPLE 60

### N¹-(2-Benzylaminocarbonylacetyl)-N⁴-[N-(tert-butoxycarbonyl)-L-phenylalanyl-L-n-valyl]-2,2-difluoro-3-hydroxy-6-methyl-1,4-heptanediamine

The title compound was prepared in 33% yield from N-(tert-butoxycarbonyl)-L-phenylalanyl-L-n-valine and the amine of Example 59 by the procedure described in Example 5.

Rf: 0.42 (silica gel; chloroform/methanol 92/8)

MS(DCI/Cl+/NH₃): 736(MNH⁺₄, 58); 719(MH+, 100).

### EXAMPLE 61

### Nl-(2-Benzylaminocarbonylacetyl)-N4-[N-(tert-butoxycarbonyl)-L-phenylalanyl-L-n-valyl]-2,2-difluoro-6-methyl-3-oxo-1,4-heptanediamine

The title compound was prepared in 46% yield from the alcohol of Example 60 by the procedure described in Example 8.

m.p.: 81°C

Rf: 0.34 (silica gel; ethyl acetate)

MS(DCl/Cl+/NH₃): 716.5(MH+, 100).

### EXAMPLE 62

### N¹-(2-Benzylaminocarbonylpropanoyl)-N⁴-(tert-butoxycarbonyl)-2,2-difluoro-3-hydroxy-6-methyl-1,4-hep- tanediamine

A mixture of 0.157 g of N¹-(2-benzylaminocarbonylpropanoyl)-2,2-difluoro-3-hydroxy- 6-methyl-1,4-hep- tanediamine (Example 55) and ditertiobutyldicarbonate (0.088 g) in anhydrous tetrahydrofuran (5 mL) was stirred at room temperature for 15 hours. Removal of the solvent in vacuo and chromatography (silica gel; ethyl acetate: cyclohexane 3/7) yielded 0.140 g of the title compound (72% yield).

Rf: 0.50 (silica gel; ethyl acetate)

MS(DCI/CI+/NH₃): 503(MNH⁺ , 60); 486(MH+, 100).

### EXAMPLE 63

### N¹-(2-Benzylaminocarbonylpropanoyl)-N⁴-(tert-butoxycarbonyl)-2,2-difluoro-6-methyl-3-oxo-1,4-heptane- diamine

The title compound was prepared in 63% yield from the alcohol of Example 62 by the procedure described in Example 8.

Rf: 0.60 (silica gel; ethyl acetate)

MS(DCI/CI+/NH3): 501(MNH⁺₄, 100); 484(MH+, 45).

### EXAMPLE 64

### N^{l-}(2-Benzylaminocarbonylpropanoyl)-2,2-difluoro-6-methyl-3-oxo-1,4-heptanediamine, hydrochloride

A mixture of 0.085 g of ketone of Example 63 and a saturated solution of hydrogen chloride in diethyl ether (6 mL) was stirred at room temperature for 15 hours. A white precipitate formed during that time. The solid was filtered off, rinsed thoroughly with pentane and dried in high vacuo.

Rf: 0.46 (silica gel; AcOH/BuOH/H₂0, 2:6:2)

MS(DCI/CI+/NH₃): 384(MH+, 100); 344(74).

### EXAMPLE 65

### N ¹-(2-Benzylaminocarbonyl-3-methylbutanoyl)-N⁴-(tert-butoxycarbonyl-2,2-difluoro-3-hydroxy-6-methyl-1,4-heptanediamine

The title compound was prepared In 650/0 yield from the amine of Example 32 by the procedure described in Example 62.

Rf: 0.62 (silica gel; ethyl acetate)

MS(DCl/Cl+/NH₃): 531(MNH⁺₄, 10); 514(MH+, 100).

### EXAMPLE 66

### N¹-(2-Benzylaminocarbonyl-3-methylbutanoyl)-N⁴-(tert-butoxycarbonyl-2,2-difluoro-6-methyl-3-oxo-1,4-hep- tanediamine

The title compound was prepared in 79% yield from the alcohol of Example 65 by the procedure described in Example 8.

Rf: 0.75 (silica gel; ethyl acetate)

MS(DCl/Cl+/NH₃): 529(MNH⁺₄, 70); 512(MH+, 100); 489(14).

### EXAMPLE 67

### N¹-(2-Benzylaminocarbonyl-3-methylbutanoyl)-N⁴-(tert-butoxycarbonyl-2,2-difluoro-6-methyl-3-oxo-1,4-hep- tanediamine, hydrochloride

The title compound was prepared in 94% yield from the ketone of Example 66 by the procedure described in Example 64.

m.p.: 110°C (decomposition)

Rf: 0.65 (silica gel; AcOH/BuOH/H₂0, 2:6:2)

MS(DCl/Cl+/NH₃): 412(MH+, 75); 372(45); 103 (100)

Analysis calculated for C₂₁ H₃₂ N₃ 0₃ F₂ Cl, H₂0: C%: 54.13; H^{O}/o: 7.35; N%: 9.02. Found: C%: 54.55; HO/o: 7.37; N%: 8.76.

### EXAMPLE 68

### N⁴-Benzyloxycarbonyl-2,2-difluoro-3-hydroxy-6-methyl-N¹-(2R-phenylacetylaminopropanoyl)-1,4-heptane- diamine

The title compound was prepared in 670/o yield from 2R-phenylacetylaminopropanoic acid and the amine of Example 4 by the procedure described in Example 9.

m.p.: 146°C

MS(DCl/Cl+/NH₃): 537(MNH⁺₄, 100); 520(MH+, 42); 386(20); 153(55)

Analysis calculated for C₂₇ H₃₅ N₃ Os F₂: C%: 62.41; H%: 6.79; Nolo: 8.09. Found: C%: 62.34; N%: 6.83; N%: 7.93.

### EXAMPLE 69

### 2,2-Difluoro-3-hydroxy-6-methyl-N¹-(2R-phenylacetylamlnopropanoyl)-1,4-heptanediamine

The title compound was prepared in 95% yield from the carbamate of Example 68 by the procedure described in Example 6.

### EXAMPLE 70

### N⁴-(tert-Butoxycarbonyl)-2,2-difluoro-3-hydroxy-6-methyl-N¹-(2R-phenylacetylaminopropanoyi)-1,4-heptane- diamine

The title compound was prepared in 78% yield from the amine of Example 69 by the procedure described in Example 62.

Rf: 0.50 (silica gel; ethyl acetate)

MS(DCl/Cl+/NH₃): 503(MNH⁺₄, 93); 486(MH₊, 100);.

### EXAMPLE 71

### N⁴-(tert-Butoxycarbonyl)-2,2-difluoro-6-methyl-N¹-(2R-phenylacetylaminopropanoyl)-3-oxo-1,4-heptane- diamine

The title compound was prepared in 960/o yield from the alcohol of Example 70 by the procedure described in Example 8.

Rf: 0.60 (silica gel; ethyl acetate)

MS(DCl/Cl+/NH₃): 501(MNH⁺₄, 55); 484(MH+, 74); 466(18); 445(44); 428(100).

### EXAMPLE 72

### 2,2-Difluoro-6-methyl-N¹-(2R-phenylacetylaminopropanoyl)-3-oxo-1,4-heptanediamine, hydrochloride

The title compound was prepared in 91% yield from the ketone of Example 71 by the procedure described in Example 64.

m.p.: 110°C (decomposition)

Rf: 0.59 (silica gel; AcOH/BuOH/H₂0, 2:6:2)

MS(DCI/Cl+/NH₃): 384(MH₊, 100); 344(34); 153(85); 103(85).

### EXAMPLE 73

### N⁴-Benzyloxycarbonyl-2,2-difluoro-3-hydroxy-6-methyl-N¹-(3-methylbutanoyl)-1,4-heptanediamine

The title compound was prepared in 86⁰/₀ yield from isovaleric acid and the amine of Example 4 by the procedure described in Example 5.

Rf: 0.33 (silica gel; ethyl acetate/cyclohexane 1:1)

MS(DCI/CI+/NHs): 432(MNH⁺₄, 100); 415(MH+, 57); 281(70).

### EXAMPLE 74

### 2,2-Difluaro-3-hydroxy-6-methyl-N¹-(3-methylbutanoyl)-1,4-heptanediamine

The title compound was prepared in 89% yield from the carbamate of Example 73 by the procedure described in Example 6.

### EXAMPLE 75

### N4-[N-(tert-Butoxycarbonyl)-L-phenylalanyl-L-n-valyl]-2,2-difluoro-3-hydroxy-6-methyl-N1-(3-methylbuta- noyl)-1,4-heptanediamine

The title compound was prepared in 82% yield from (N-(tert-butoxycarbonyl)-L-phenylalanyl-L-n-valine and the amine of Example 74 by the procedure described in Example 9.

Rf: 0.41 (silica gel; chloroform/methanol 92:8)

MS(DCl/Cl+/NH₃): 644(MNH⁺₄, 61); 627(MH+, 100).

### EXAMPLE 76

### N⁴-[N-(tert-Butoxycarbonyl)-L-phenylalanyl-L-n-valyl]-2,2-difluoro-6-methyl-N¹-(3-methylbutanoyl)-3-oxo-1,4-heptanediamine

The title compound was prepared in 60% yield from the alcohol of Example 75 by the procedure described in Example 8.

Rf: 0.49 (silica gel; chloroform/methanol 92:8)

MS(DCl/Cl+/NH₃): 643(MNH§ , 100); 625(MH+, 35).

Analysis calculated for: C₃₂ Hso N₄ O₆ F₂: C%: 61.52; H%: 8.07; N%: 8.97. Found: C%: 61.56; H%: 8.35; N%: 8.81.

### EXAMPLE 77

### N⁴-Benzyloxycarbonyl-2,2-difluoro-3-hydroxy-5-phenyl-1,4-pentanediamine

The trifluoroacetic acid salt of the title compound was prepared in quantitative yield from the carbamate of Example 15 by the procedure described in Example 4. An ethereal solution of this TFA salt was washed with saturated sodium bicarbonate (3 times) and dried over anhydrous magnesium sulphate. Filtration and removal of the solvent in vacuo yielded the title compound in 88% yield as a white solid.

MS(DCl/Cl+/NH₃); 382(MNH⁺₄, 3); 365(MH+, 100).

### EXAMPLE 78

### N¹-Acetyl-N⁴-benzyloxoycarbonyl-2,2-difluoro-3-hydroxy-5-phenyl-1,4-pentanediamine

The title compound was prepared in 70% yield from acetic anhydride and the amine of Example 77 by the procedure described in Example 17.

Rf: 0.39 (silica gel; ethyl acetate)

MS(DCl/Cl+/NH₃): 424(MNH⁺₄, 100); 407(MH+, 23); 273(62).

### EXAMPLE 79

### N¹-Acetyl-2,2-difluoro-3-hydroxy-5-phenyl-1,4-pentanediamine

The title compound was prepared in 840/₀ yield from the carbamate of Example 78 by the procedure described in Example 6.

### EXAMPLE 80

### N¹-Acetyl-N⁴-(N-acetyl-L-Leucyl)-2,2-difluoro-3-hydroxy-5-phenyl-1,4-pentanediamine

The title compound was prepared in 72% yield from N-acetyl-L-leucine and the amine of Example 79 by the procedure described in Example 9.

Rf: 0.17 (silica gel; chloroform/methanol 92:8)

MS(DCl/Cl+/NH₃): 445(MNH⁺₄, 100); 428(MH+, 54.

Analysis calculated for: C₂₁ H₃₁ N₃ 0₄ F₂; C%: 59.00; H%: 7.31; N%: 9.83. Found: C%: 58.98; H%: 7.24; N%: 9.42.

### EXAMPLE 81

### N¹-Acetyl-N⁴-(N-acetyl-L-Leucyl)-2,2-dffluoro-3-oxo-5-phenyl-1,4-pentanediamine

The title compound was prepared in 73% yield from the alcohol of Example 80 by the procedure described in Example 8.

Rf: (silica gel; chloroform/methanol 92:8)

MS(DCl/Cl+/NH₃): 443(MNH⁺₄, 100); 426(MH+, 23).

Analysis calculated for C₂₁ H₂₉ N₃ 0₄ F₂: C%: 59.28; H%: 6.87; N%: 9.88. Found: C%: 59.51; N%: 7.02; N%: 9.65.

### EXAMPLE 82

### N⁴-Benzyloxycarbonyl-2,2-difiuoro-3-hydroxy-5-methyl-1,4-hexanediamine, trifluoroacetate

The title compound was prepared in 66% yield from the carbamate of Example 23 by the procedure described in Example 77.

### EXAMPLE 83

### N¹-Acetyl-N4-benzyloxycarbonyl-2,2-dffluoro-3-hydroxy-5-methyl-1,4-hexanediamine

The title compound was prepared in 830/o yield from the amine of Example 82 and acetic anhydride by the procedure described in Example 17.

### EXAMPLE 84

### N¹-Acetyl-2,2-difluoro-3-hydroxy-5-methyl-1,4-hexanediamine

The title compound was prepared in 89% yield from the carbamate of Example 83 by the procedure described in Example 6.

### EXAMPLE 85

### N¹-Acetyl-2,3-difluoro-3-hydroxy-N⁴-(methoxysuccinyl-L-alanyl-L-alanyl-L-prolyl)-5-methyl-1,4-hexanediamine

The title compound was prepared in 43% yield from methoxysuccinyl-L-alanyl-L-alanyl-L-proline and the amine of Example 84 by the procedure described in Example 5.

Rf: 0.19 (silica gel: chloroform/methanol 92:8)

MS(DCl/Cl+/NH₃): 596(MH+, 23); 563(100); 546(41).

### EXAMPLE 86

### N¹-Acetyl-2,2-difluoro-N4-(methoxysuccinyl-L-alanyl-L-alanyl-L-prolyl)-5-methyl-3-oxo-1,4-Mexanediamine

The title compound was prepared in 60% yield from the carbamate of Example 85 by the procedure described in Example 8.

Rf: 0.23 (silica gel; chloroform/methanol 92:8)

MS(DCI/CI+/NH₃): 593(MNH⁺₄, 100); 576(MH₊, 52).

Analysis calculated for C₂₅ H₃₉ N₅ 0₈ F₂; C%: 52.17; H%. 6.83; N%: 12.17. Found: G%: 52.54; H%: 6.95; N%: 11.58.

### EXAMPLE 87

### N⁴-Benzyloxycarbonyl-N¹-(2-methylpropyloxycarbonyl)-2,2-difluoro-hydroxy-6-methyl-1,4-heptanediamirbe

The title compound was prepared in 89⁰/₀ yield from isobutylchloroformate and the amine of Example 4 by the procedure described in Example 17.

Rf: 0.45 (silica gel; ethyl acetate/cyclohexane 1:1)

MS(DCI/CI+/NH₃): 448(MNH⁺₄, 100); 431(MH+, 28); 340(15); 297(57).

### EXAMPLE 88

### 2,2-Difluoro-3-hydroxy-6-methyl-N ¹⁻(2-methylpropyloxycarbonyl)₋1,4-heptanediamine

The title compound was prepared in 93% yield from the carbamate of Example 87 by the procedure described in Example 6.

### EXAMPLE 89

### N⁴-[N-(tert-Butoxycarbonyl)-L-phenylalanyl-L-n-valyl]-2,2-difluoro-3-hydroxy-6-methyl-N¹-(2-methylpropylox- ycarbonyl)-1,4-heptanediamine

The title compound was prepared in 620/o yield from N-(tert-butoxycarbonyl)-L-phenylalanyl-L-n-valine and the amine of Example 88 by the procedure described in Example 5.

Rf: 0.60 (silica gel; chloroform/methanol 92:8)

MS(DCI/Cl+/NH₃): 660(MNH⁺₄, 30); 643(MH⁺, 100).

### EXAMPLE 90

### N⁴-[N-(tert-Butoxycarbonyl)-L-phenylalanyl-L-n-valyl]-2,2-difluoro-6-methyl-N¹-(2-methylpropyloxycarbo- nyl)-3-oxol,4-heptanediamine

The title compound was prepared in 84% yield from the alcohol of Example 89 by the procedure described in Example 8.

m.p.: 131°C

Rf: 0.44 (silica gel; ethyl acetate/cyclohexane 1:1)

MS(DCI/CI+/NH₃): 658(MNH( , 100); 641(MH+, 98)

Analysis calculated for C₃₂ H₅ₒ N₄ 0₇ F₂: C%: 59.98; H%: 7.86; N%: 8.74. Found: C%: 60.40; H%: 8.11; N%: 8.39.

### EXAMPLE 91

### N⁴-Benzoyl-N¹-(2-benzyloxycarbonylacetyl)-2,2-difluoro-3-hydroxy-5-phenyl-1,4-pentanediamine

The title compound was prepared in 42% yield from 2-benzyloxycarbonylacetic acid and the amine of Example 18 by the procedure described in Example 9.

Rf: 0.17 (silica gel; ethyl acetate/cyclohexane 1:1)

MS(CI/NH₃): 511(MH+, 100).

### EXAMPLE 92

### N⁴-Benzoyl-N¹-(2-benzy(oxycarbonylacetyl)-2,2-diftuoro-3-oxo-5-phenyl-1,4-pentanediamine

The title compound was prepared in 50% yield from the alcohol of Example 91 by the procedure described in Example 8.

### EXAMPLE 93

### N⁴-Benzoyl-N¹-(2-carboxyacetyl)-2,2-difluoro-3-oxo-5-phenyl-1,4-pentanediamine

The title compound was prepared in quantitative yield from the ester of Example 92 by the procedure described in Example 6.

### EXAMPLE 94

### N-Benzyloxycarbonyl-3-cyclohexylalanine

To a solution of 3-cyclohexylalanine, hydrochloride (4.75 g, 22.8 mmol) in 2N sodium hydroxide (11.4 mL) were added at 0°C, simultaneously, a solution of benzylchloroformate (3.2 mL, 36 mmol) in tetrahydrofuran (10 mL) and 2N sodium hydroxide (11.4 mL). (The pH of the mixture was maintained around 9-10 by addition of 2N sodium hydroxide.) The mixture was stirred for 1.5 hours. The solution was washed with diethyl ether (3 x 20 mL). The aqueous phase was acidified to pH 2 with 3N aqueous hydrochloric acid and extracted with ethyl acetate (3 x 50 mL). The combined organic layers were dried over anhydrous magnesium sulphate. Filtration and removal of the solvent in vacuo left 4.80 g of the expected product (yellow oil, 69⁰/₀ yield).

Rf: 0.75 (silica gel; AcOH/BuOH/H₂0 2:6:2).

### EXAMPLE 95

### 2-Benzyloxycarbonylamino-3-cyclohexyl-N,O-dimethyl propanehydroxamate

To a solution of N-benzyloxycarbonyl-3-cyclohexylalanine (4.60 g, 15 mmol) in anhydrous methylene chloride (60 mL) were added, at 0°C, dicyclohexylcarbodiimide (3.09 g, 15 mmol) and 1-hydroxybenzotriazole- hydrate (2.29 g, 15 mmol). After stirring for 0.25 hours at 0° C, N,O,-dimethylhydroxylamine hydrochloride (1.46 g, 15 mmol) and N-methylmorpholine (1.51 g, 15 mmol) were added to the mixture. The mixture was stirred for 20 hours while the temperature was allowed to rise to room temperature. The precipitate was filtered off. The solvent was removed in vacuo and the mixture was purified by chromatography (silica gel; ethyl acetate/cyclohexane 2:8) yielding 3.60 g of the expected hydroxamate (69% yield).

Rf: 0.38 (silica gel; ethyl acetate/cyclohexane 1:1, UV, 1₂).

### EXAMPLE 96

### N-Benzyloxycarbonyl-3-cyclohexylalaninal

A mixture of 2-benzyloxycarbonylamino-3-cyclohexyl-N,O-dimethylpropanehydroxamate (3.58 g, 10.3 mmol), and lithium aluminiumhydride (0.44g, 11.6 mmol) in anhydrous diethyl ether (100 mL) was stirred at 0°C for 1 hour. 1 M Potassium hydrogenosulphate (25 mL) was added. The mixture was stirred for 0.5 hour and extracted with diethyl ether (2 x 25 mL).

The combined organic layers were washed with 2N HCI (3 x 20 mL), water (1 x 20 mL), a saturated solution of sodium bicarbonate (1 x 20 mL), brine (20 mL) and dried over anhydrous magnesium sulphate. Filtration and removal of the solvent in vacuo left 2.52 g of the expected aldehyde (85%, yellowish oil) used in the next step without further purification.

### EXAMPLE 97

### 4-Benzyloxycarbonylamino-5-cyclohexyl-2,2-difluoro-3-hydroxypentanoic acid, ethyl ester

The title compound was prepared in 37% yield from N-benzyloxycarbonyl-3-cyclohexylalaninal, ethyl bromodlfluoroacetate and zinc by the procedure described in Example 1.

Rf: 0.57 (silica gel; ethyl acetate/cyclohexane 1:1).

### EXAMPLE 98

### 4-Benzyloxycarbonylamino-5-cyclohexyl-2,2-difluoro-3-hydroxypentanamide

The title compound was prepared in 97% yield from the ester of Example 97 by the procedure described in Example 2.

Rf: 0.53 (silica gel; ethyl acetate)

MS(DCI/CI+/NH3): 402(MNH4 , 86); 385(MH+, 13); 294(23); 169(40); 126(100).

### EXAMPLE 99

### N⁴-Benzyloxycarbonyl-N¹-(tert-butoxycarbonyl)-5-cyclohexyl-2,2-difluoro-3-hydroxy-1,4-pentanediamine

The title compound was prepared in 51% yield from the amide of Example 98 by the procedure described In Example 3.

Rf: 0.59 (silica gel; ethyl acetate/cyclohexane 1:1).

### EXAMPLE 100

### N⁴⁻Benzyloxycarbonyl-5-cyclohexyl-2,2-difluoro-3-hydroxy-1,4-pentanediamine

The title compound was prepared in 60% yield from the carbamate of Example 99 by the procedure described in Example 77.

### EXAMPLE 101

### N¹-(2-Benzylaminocarbonyl-3-methylbutanoyl)-N⁴-benzyloxycarbonyl-5-cyclohexyl-2,2-difluoro-3-hydroxy-1,4-pentanediamine

The title compound was prepared in 58% yield from 2-benzylaminocarbonyl-3-methylbutanoic acid and the amine of Example 100 by the procedure described in Example 9.

Rf: 0.60 (silica gel; ethyl acetate)

Analysis calculated for C₃₂ H₄₃ N₃ O₅ F₂: C^{O}/o: 65.40; H%: 7.37; N%: 7.15. Found: C^{O}/o: 65.01; H^{O}/o: 7.42; N%: 7.06.

### EXAMPLE 102

### N¹-(2-Benzylaminocarbonyl-3-methylbutanoyl)-5-cyclohexyl-2,2-difluoro-3-hydroxy-1,4-pentanediamine

The title compound was prepared in 82% yield from the carbamate of Example 101 by the procedure described in Example 6.

### EXAMPLE 103

### N¹-(2-Benzylaminocarbonyl-3-methylbutanoyl)-N⁴-[N-(tert-butoxycarbonyl)-L-phenylalanyl-L-n-valyl]-5-cyclohexyl-2,2-difluoro-3-hydroxy-1,4-pentanediamine

The title compound was prepared in 600/o yield from N-(tert-butoxycarbonyl)-L-phenylalanyl-L-n-valine and the amine of Example 102 by the procedure described in Example 5.

Rf: 0.46 (silica gel; chloroform/methanol 92:8)

MS(DCI.CI+.NH3): 817/ 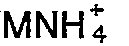 ₁₀)_{; 800}(_{MH+}, ₁₀₀)_{; 700}(₆₀).

Analysis calculated for: C₄₃ H₆₃ N₅ 0₇ F₂: C%: 64.56; H%: 7.94; N%: 8.75. Found: C%: 64.47; H%: 8.13; N%: 8.58.

### EXAMPLE 104

### N¹-(2-Benzylaminocarbonyl-3-methylbutanoyl)-N⁴-[N-(tert-butoxycarbonyl)-L-phenylalanyl-L-n-valyl]-5-cyclohexyl-2,2-difluoro-3-oxo-1,4-pentanediamine

The title compound was prepared in 640/₀ yield from the alcohol of Example 103 by the procedure described in Example 8.

Rf: 0.53 (silica gel; chloroform/methanol 92:8)

MS(DCI/CI+/NH₃): 815( 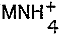 , 10); 798(MH₊, 100).

### EXAMPLE 105

### Ethyl 4-benzyloxycarbonylamino-2,2-difluoro-3-hydroxy butanoate

The title compound was prepared in 33% yield from N-benzyloxycarbonylglycinal, ethyl bromodifluoroacetate and zinc by the procedure described in Example 1.

Rf: 0.45 (silica gel; ethyl acetate/cyclohexane 1:1).

### EXAMPLE 106

### 4-Benzyloxycarbonylamino-2,2-difluoro-3-hydroxy butanamide

The title compound was prepared in 95% yield from the ester of Example 105 by the procedure described in Example 2.

Rf: 0.49 (silica gel; ethyl acetate/cyclohexane 1:1).

### EXAMPLE 107

### N⁴-Benzyloxycarbonyl-N¹-(tert-butoxycarbonyl)-2,2-difluoro-3-hydroxy-1,4-butanediamine

The title compound was prepared in 48% yield from the amide of Example 106 by the procedure described in Example 3.

Rf: 0.42 (silica gel; ethyl acetate/cyclohexane 1:1)

MS(DCI/CI+/NH₃): 392(MNH⁺ , 59); 375(MH+, 20); 258(15); 241(100).

### EXAMPLE 108

### Ethyl 4-benzyloxycarbonylamino-2,2-difluoro-3-hydroxy pentanoate

The title compound was prepared in 50% yield from N-benzyloxycarbonylalaninal, ethyl bromodifluoroacetate and zinc by the procedure described in Example 1.

Rf: 0.49 (silica gel; ethyl acetate/cyclohexane 1:1).

### EXAMPLE 109

### 4-Benzyloxycarbonylamino-2,2-difluoro-3-hydroxy pentanamide

The title compound was prepared in 90% yield from the ester of Example 108 by the procedure described in

### Example 2.

Rf: 0.50 (silica gel; ethyl acetate/cyclohexane 1:1)

MS(DCI/CI+/NH₃): 320(MNH⁺ , 100); 303(MH+, 13); 212(19); 169(100).

### EXAMPLE 110

### N⁴-Benzyloxycarbonyl-N¹-(tert-butoxycarbonyl)-2,2-difluoro-3-hydroxy-1,4-pentanediamine

The title compound was prepared in 53% yield from the amide of Example 109 by the procedure described in Example 3.

Rf: 0.47 (silica gel; ethyl acetate/cyclohexane 1:1)

MS(DCI/CI+/NH₃): 406(MNH+ , 94); 389(MH+, 23); 298(20); 255(100).

### EXAMPLE 111

### N⁴-Benzyloxycarbonyl-5-cyclohexyl-2,2-difluoro-3-hydroxy-N¹-(3-methylbutanoyl)-1,4-pentanediamine

The title compound was prepared in 60% yield from isovaleric acid and the amine of Example 100 by the procedure described in Example 5.

Rf: 0.34 (silica gel; ethyl acetate/cyclohexane 1:1)

MS(DCI/CI+/NH₃): 472( 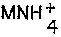 + 4, 63); 455(MH+, 34); 321(100).

### EXAMPLE 112

### 5-Cyclohexyl-2,2-difluoro-3-hydroxy-N¹-(3-methylbutanoyl)-1,4-pentanediamine

The title compound was prepared in 69% yield from the carbamate of Example 111 by the procedure described in Example 6.

### EXAMPLE 113

### N⁴-[N-(tert-Butoxycarbonyl)-L-phenylalanyl-L-n-valyl]-5-cyclohexyl-2,2-difluoro-3-hydroxy-N¹-(3-methylbuta- noyl)-₁,₄-pentanediamine

The title compound was prepared in 780/o yield from N-(tert-butoxycarbonyl)-L-phenylalanyl-L-n-valine and the amine of Example 112 by the procedure described in Example 5.

Rf: 0.18 (silica gel; ethyl acetate/cyclohexane 1:1)

MS(DCI/CI+/NH3): 684(MNH⁺ , 29); 667(MH+, 100);

### EXAMPLE 114

### N⁴-[N-(tert-Butoxycarbonyl)-L-phenylalanyl-L-n-valyl-5-cyclohexyl-2,2-difluoro-N¹-(3-methylbutanoyl)-3-oxo-1,4-pentanediamine

The title compound was prepared in 740/₀ yield from the alcohol of Example 113 by the procedure described in Example 8.

Rf: 0.41 (silica gel; ethyl acetate/cyclohexane 1:1)

MS(DCI/CI+/NH₃): 682( 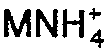 , 5); 665(MH+, 18); 364(100)

Analysis calculated for C₃₅ N₅₄ N₄ O₆ F₂: C%: 63.23; H%: 8.19; N%: 8.43. Found C%: 62.78; H%: 8.27; N%: 8.12.

### EXAMPLE 115

### N⁴-Benzyloxycarbonyl-2,2-difluoro-3-hydroxy-6-methyl-N¹-[2-(2-methylpropyl)-5-phenylpentanoyl]-1,4-hep- tanediamine

The title compound was prepared in 540/o yield from 2-(2-methylpropyl)-5-phenylpentanoyl chloride and the amine of Example 4 by the procedure described in Example 17.

MS(DCI/CI+/NHs): 564( 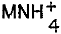 , 40); 547(MH+, 30); 413(100).

### EXAMPLE 116

### 2,2-Difluoro-3-hydroxy-6-methyl-N¹-[2-(2-methylpropyl)-5-phenylpentanoyl]-1,4-heptanediamine

The title compound was prepared in 87% yield from the carbamate of Example 115 by the procedure described in Example 6.

MS(DCI/CI+/NH3): 413(MH+).

### EXAMPLE 117

### N⁴-[N-(tert-Butoxycarbonyl)-L-phenylalanyl-L-n-valyl]-2,2-diffluoro-3-hydroxy-6-methyl-N¹-[2-(2-methylpropyl)-5-phenylpentanoyl]-1,4-heptanediamine

The title compound was prepared in 26% yield from N-(tert-butoxycarbonyl)-L-phenylalanyl-L-n-valine and the amine of Example 116 by the procedure described in Example 5.

Rf: 0.61 (silica gel; chloroform/methanol 92:8)

MS(DCI/CI+/NH3) : 776(MNH+ , 65); 759(MH+, 89); 282(85); 265(100).

### EXAMPLE 118

### N⁴⁻[N-(tert-Butoxycarbonyl)-L-phenylalanyl-L-n-valyl]-2,2-difluoro-6-methyl-N¹⁻[2-(2-methylpropyl)-5-phe- nylpentanoyl]-3-oxo-1,4-heptanediamine

The title compound was prepared in 62% yield from the alcohol of Example 117 by the procedure described in Example 8.

Rf: 0.50 (silica gel; ethyl acetate/cyclohexane 1:1)

Analysis calculated for C₄₂ H₆₂ N₄ Os F₂: C^{O}/o: 66.64; HO/o: 8.25; NO/o: 7.40. Found: C%: 66.62; HO/o: 8.52; NO/o: 6.86.

### EXAMPLE 119

### N-Benzyloxycarbonyl-4-nitrophenylalanine, methyl ester

To a mixture of N-benzyloxycarbonyl-4-nitrophenylalanine (0.3 g, 0.9 mmol) in ethyl acetate (10 mL) was added at 0°C, a 0.5M solution of diazomethane in diethyl ether until persistence of a yellow color. The mixture was stirred for 0.5 hour at 0° C. Acetic acid was added to destroy the excess of diazomethane (disappearance of the yellow color). Removal of the solvent in vacuo and purification of the crude product by chromatography (silica gel; ethyl acetate/cyclohexane 2:8) yielded 0.219 g of the title compound (700/₀ yield).

Rf: 0.38 (silica gel. ethyl acetate/cyclohexane 1:1).

### EXAMPLE 120

### N-Benzyloxycarbonyl-4-nitrophenylalaninal

To a suspension of N-benzyloxycarbonyl-4-nitrophenylalanine methyl ester (2.55 g 7.1 mmol) in a mixture of toluene and diethyl ester (60 mL, 5:1), was added dropwise at -78°C, under nitrogen, a solution of DIBAL in toluene (1M, 14.3 mL). The mixture was stirred at -78°C for 30 min. Methanol (5 mL) and a saturated aqueous solution of Rochelle's salt (30 mL) were added and the mixture was extracted with diethyl ether (2 x 100 mL). The combined organic layers were dried over anhydrous magnesium sulphate. Filtration, removal of the solvent in vacuo, and chromatography (silica gel, ethyl acetate/n hexane 2:8) yielded 1.709 g of the title compound (73% yield).

Rf: 0.18 (silica gel; ethyl acetate/cyclohexane 1:1).

### EXAMPLE 121

### 4-Benzyloxycarbonylamino-2,2-difluoro-3-hydroxy-5-(4-nitrophenyl)pentanoic acid, ethyl ester

The title compound was prepared in 30% yield from the aldehyde of Example 120, ethyl bromodifluoroacetate and zinc, by the procedure described in Example 1.

MS(DCI/CI+/NH₃): 470(MNH+ , 100); 453(MH+, 5).

### EXAMPLE 122

### 4-Benzyloxycarbonylamino-2,2-difluoro-3-hydroxy-5-(4-nitrophenyl)pentanamide

The title compound was prepared in 70% yield from the ester of Example 121 by the procedure described in Example 2.

### EXAMPLE 123

### N⁴-Benzyloxycarbonyl-N¹-tert-butoxycaronyl-2,2-difluoro-3-hydroxy-5-(4-nitrophenyl)-1,4-pentanediamine

The title compound was prepared from the amide of Example 122 by the procedure described in Example 3.

### EXAMPLE 124

### N⁴⁻Benzyloxycarbonyl-2,2-difluoro-3-hydroxy-5-(4-nitrophenyl)-1,4-pentanediamine

The title compound was prepared from the carbamate of Example 123 by the procedure described in

### Example 77.

### EXAMPLE 125

### N¹-Acetyl-N⁴-benzyloxycarbonyl-2,2-difluoro-3-hydroxy-5-(4-nitrophenyl)-1,4-pentanediamine

The title compound was prepared from the amine of Example 124 and acetic anhydride by the procedure described in Example 17.

### EXAMPLE 126

### N¹-Acetyl-N⁴-benzyloxycarbonyl-2,2-difluoro-3-hydroxy-5-(4-aminophenyl)-1,4-pentanediamine

A mixture of N¹-acetyl-N⁴-benzyloxycarbonyl-2,2-difluoro-3-hydroxy-5-(4-nitrophenyl)-1,4-pentanediamine (0.451 g, 1 mmol) and tin dichloride, 2 H₂0 (1.128 g, 5 mmol) in absolute ethanol (5 mL) was heated at reflux under nitrogen for 1 hour. The mixture was allowed to cool to room temperature and was poured into ice. The pH was made slightly basic (pH 7-8) by addition of aqueous sodium bicarbonate, and the mixture was extracted with ethyl acetate. The organic phase was washed with brine, treated with charcoal and dried over anhydrous magnesium sulphate. Filtration and removal of the solvent in vacuo yielded the title compound.

### EXAMPLE 127

### N¹-Acetyl-N⁴-benzyloxycarbonyl-5-(4-tert-butoxycarbonylaminophenyl)-2,2-difluoro-3-hydroxy-1,4-pentanediamine

A solution of amine of Example 126 (0.214 g, 0.5 mmol) and di-tert-butyldlcarbonate (0.131 g, 0.6 mmol) in tetrahydrofuran (10 mL) was heated at reflux for 15 hours, under nitrogen. Removal of the solvent in vacuo and chromatography (silica gel, ethyl acetate/cyclohexane 1:1) yielded the expected carbamate.

### EXAMPLE 128

### N¹-Acetyl-5-(4-tert-butoxycarbonylaminophenyl)-2,2-difluoro-3-hydroxy-1,4-pentanediamine

The title compound was prepared from carbamate of Example 127 by the procedure described in Example 6.

### EXAMPLE 129

### N¹-Acetyl-N⁴-benzoyl-5-(4-tert-butoxycarbonylaminophenyl)-2,2-difluoro-3-hydroxy-1,4-pentanediamine

The title compound was prepared from the amine of Example 128 and benzoyl chloride by the procedure described in Example 17.

### EXAMPLE 130

### N¹-Acetyl-N⁴-benzoyl-5-(4-tert-butoxycarbonylaminophenyl)-2,2-difluoro-3-oxo-1,4-pentanediamine

The title compound was prepared from the alcohol of Example 129 by the procedure described inm Example 8.

### EXAMPLE 131

### N¹-Acetyl-5-(4-aminophenyl)N⁴-benzoyl-2,2-difluoro-3-oxo-1,4-pentanediamine

The title compound was prepared from the carbamate of Example 130 by the procedure described in Example 77.

### EXAMPLE 132

### N¹-Acetyl-N⁴-benzoyl-5[4-(N',N"-bis(tert-butoxycarbonyl)guanidino]phenyl]-2,2-difluoro-3-oxo-1,4-pentanediamine

A mixture of N¹-acetyl-5-(4-aminophenyl)-N⁴-benzoyl-2,2-difluoro-3-oxo-1,4-pentanediamine (0.195 g, 0.5 mmol) and N,N-bis(tert-butoxycarbonyl)-S-methylisothiourea (0.174 g, 0.6 mmol) In tetrahydrofuran (10 mL) was heated at 55° C for 15 hours. Removal of the solvent in vacuo left a solid residue. The residue was treated with 5% sodium bicarbonate and extracted with chloroform (2 x 20 mL). The combined organic layers were washed with water and the product was purified by chromatography (silica gel, chloroform/methanol 2:98) to yield the title compound.

### EXAMPLE 133

### N¹-Acetyl-N⁴-benzoyl-5-(4-guanidinophenyl)-2,2-difluoro-3-oxo-1,4-pentanediamine, bistrifluoroacetate

The title compound was prepared from the carbamate of Example 132 by the procedure described in Example 4.

### EXAMPLE 134

### e-N-Benzyloxycarbonyl-α-N-4-nitrobenzyloxycarbonyllysine

To a solution of e-N-benzyloxycarbonyllysine (2.80 g, 10 mmol) in a mixture of tetrahydrofuran (25 mL) and 0.5N sodium hydroxide (50 mL) was added dropwise, at 0°C, a solution of 4-nitro-benzylchloroformate (2.695 g) in tetrahydrofuran (25 mL). After completion of the addition, the temperature was allowed to rise to room temperature, and the mixture was stirred for 2 hours at that temperature. The mixture was diluted with water and washed with diethyl ether (2 x 50 mL). The aqueous phase was acidified (pH 2) and extracted with chloroform (3 x 80 mL). The combined organic extracts were dried over anhydrous magnesium sulphate. Filtration and removal of the solvent in vacuo yielded 4.042 g of the title compound (88% yield).

Rf: 0.85 (silica gel; AcOH/BuOH/H₂0 2:6:2).

MS(DCI/CI+/NH₃): 477(MNH+ , 80); 460(MH+, 15); 324(18); 281(85); 250(20); 237(52); 235(100); 218(45).

### EXAMPLE 135

### ε-N-Benzyloxycarbonyl-α-N-4-nitrobenzyloxycarbonyllysine, methyl ester

The title compound was prepared in 90% yield from the acid of Example 134 by the procedure described in Example 119.

m.p.: 74-75°C

Rf: 0.19 (silica gel; ethyl acetate/cyclohexane 1:1)

MS(DCI/CI+/NHs): 491( 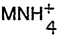 , 44); 474(MH+, 7); 295(100).

### EXAMPLE 136

### s-N-Benzyloxycarbonyl-a-N-4-nitrobenzyloxycarbonyllysinal

The title compound was prepared in 760/0 yield from the ester of Example 135 by the procedure described in Example 120.

Rf: 0.11 (silica gel. ethyl acetate/cyclohexane 1:1).

### EXAMPLE 137

### 8-Benzyloxycarbonylamino-2,2-difluoro-3-hydroxy-4-(4-nitrobenzyloxycarbonylamino)-octanoic acid, ethyl ester

The title compound was prepared in 30% yield from the aldehyde of Example 136 by the procedure described in Example 1.

Rf: 0.56 (silica gel. ethyl acetate).

MS(DCI/CI+/NHs): 585(MNH+ , 100); 568(MH+, 19); 450(23); 389(65); 255(45); 235(63).

### EXAMPLE 138

### 8-Benzyloxycarbonylamino-2,2-difluoro-3-hydroxy-4-(4-nitrobenzyloxycarbonylamino)-octanamide

The title compound was prepared in quantitative yield from the ester of Example 137 by the procedure described in Example 2.

Rf: 0.25 (silica gel; ethyl acetate)

MS(DCI/CI+/NH₃): 556( 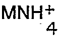 , 54); 539(MH+, 4); 421(13); 403(46); 360(41); 139(38); 106(100).

### EXAMPLE 139

### N⁸⁻Benzyloxycarbonyl-Nⁱ⁻(tert-butoxycarbonyl)-2,2-difluoro-3-hydroxy-N⁴⁻(4-nitrobenzyloxycarbonyl)-1,4,8-octanetriamine

The title compound was prepared in 32% yield from the amide of Example 138 by the procedure described in Example 3.

MS(DCI/CI+/NH₃): 642( 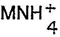 , 100); 625(MH+, 10); 525(20); 507(20); 490(8); 446(28); 312(45).

### EXAMPLE 140

### N⁸-Benzyloxycarbonyl-2,2-difluoro-3-hydroxy-N⁴-(4-nitrobenzyloxycarbonyl)-1,4,8-octanetriamine

The title compound was prepared from the carbamate of Example 139 by the procedure described in

### Example 77.

### EXAMPLE 141

### N¹-Acetyl-N⁸-benzyloxycarbonyl-2,2-difluoro-3-hydroxy-N⁴-(4-nitrobenzyloxycarbonyl-1,4,8-octanetriamine

The title compound was prepared from the amine of Example 140 by the procedure described in Example 17.

### EXAMPLE 142

### N¹-Acetyl-N⁸-benzyloxycarbonyl-2,2-difluoro-3-hydroxy-1,4,8-octanetriamine

A midture of N^{l-}acetyl-N⁸⁻benzyloxycarbonyl-2,2-difluoro-3-hydroxy-N⁴⁻(4-nitrobenzyloxycarbonyl)-1,4,8-octanetriamine (0.273 g, 0.5 mmol) and tin dichloride, 2 H₂0 (0.564 g, 2.5 mmol) in absolute ethanol (5 mL) was heated at reflux under nitrogen for 1 hour. The mixture was allowed to cool to room temperature and was poured into ice. The pH was made basic (9-10) and the mixture was extracted with ethyl acetate. The organic phase was washed with brine, treated with charcoal and dried over anhydrous magnesium sulphate. Filtration and removal of the solvent in vacuo yielded the title compound.

### EXAMPLE 143

### N¹-Acetyl-N⁸-benzyloxycarbonyl-N⁴-[N-(tert-butoxycarbonyl)-D-phenylalanyl-L-prolyl]-2,2-dffluoro-3-hydroxy-1,4,8-octanetriamine

The title compound was prepared from the amine of Example 142 and N-(tert-butoxycarbonyl)-D-phenylala- nylproline by the procedure described in Example 5.

### EXAMPLE 144

### N¹-Acetyl-N⁴-[N-(tert-butoxycarbonyl)-D-phenylalanyl-L-prolyl]-2,2-diffuoro-3-hydroxy-1,4,8-octanetriamine

The title compound was prepared from the carbamate of Example 143 by the procedure described in Example 6.

### EXAMPLE 145

### N 1-Acetyl-N8-(tert-butoxycarbonyl)-N4-[N-(tert-butoxycarbonyl)-D-phenylalanyl-L-prolyl]-2,2-difluoro-3-hydroxy-1,4,8-octane-triamine

The title compound was prepared from the amine of Example 144 and dl-tert-butyldicarbonate by the procedure described in Example 127.

### EXAMPLE 146

### N¹-Acetyl-N⁸-(tert-butoxycarbonyl)-N⁴-[N-(tert-butoxycarbonyl)-D-phenylalanyl-L-prolyl]-2,2-difluoro-3-oxo-1,4,8-octanetriamine

The title compound was prepared from the alcohol of Example 145 by the procedure described in Example 8.

### EXAMPLE 147

### N¹-Acetyl-N⁴-(D-phenylalanyl-L-prolyl)-2,2-difluoro-3-oxo-1,4,8-octanetriamine, bishydrochloride

The title compound was prepared from the ketone of Example 146 by the procedure described in Example 64.

The foregoing describes in detail the generic and specific aspects of the scope of the invention as well as the manner of making and using the invention. In addition thereto, although such procedures are known in the art, references setting forth state of the art procedures by which the compounds may be evaluated for their biochemical effects are also included herein.

For example, human elastase is assayed in vitro using chromophoric peptides, succinylalanylalanylalanyl- p-nitroanilide (A1), methoxysuccinylalanylalanylprolylvalyl-p-nitroanilide (A2), and others, all of which are available commercially. The assay buffer, pH 8.0, and assay techniques are similar to those described by Lottenberg, et al. (A3, A4). Enzyme is purified from human sputum (A5), although recently it has become commercially available. Kinetic characterization of immediate inhibitors is by means of the Dixon plot (A6), whereas the characterization of slow- and/or tight-binding inhibitors used data analysis techniques reviewed by Williams and Morrison (A7).

Similarly, the other proteases are assayed and effects of inhibitors are assessed in vitro by similar spectroscopic techniques: cathepsin G (A2); thrombin (A3); chymotrypsin (A8); trypsin (A9); plasmin (A3); Cl esterase (A10); urokinase (A3); plasminogen activator (A11); acrosin (A12); beta-lactamase (A13); cathepsin B (A14); pepsin (A15); cathepsin D (A16) and leucine aminopeptidase (A17). Pseudomonas elastase was measured in a coupled assay procedure using a human elastase substrate and microsomal aminopeptidase.

Radiometric assays of angiotensin I-converting enzyme and enkephalinase and their inhibitors were based on the procedure of Ryan (A18) and used tritiated substrated purchased from Ventrex Laboratories, Inc. Radioimmunoassay was used for studies with renin (A19). C3-convertase was measured as described by Tack, et al. (A20).

The individual assay references are elaborated upon by the following:
A1. The synthesis and analytical use of a highly sensitive and convenient substrate of elastase. J. Bieth, B. Spiess and C.G. Wermuth, Biochemical Medicine, 11 (1974) 350-375.
A2. Mapping the extended substrate binding site of cathepsin G and human leukocyte elastase. Studies with peptide substrates related to the alpha 1-protease inhibitor reactive site. K. Nakajima, J.C. Powers, B.M. Ashe and M. Zimmerman, The Journal of Biological Chemistry, 254 (1979) 4027-4032.
A3. Assay of coagulation proteases using peptide chromogenic and fluorogenic substrates. R. Lottenberg, U. Christensen, C.M. Jackson and P.L. Coleman, in Methods In Enzymology (L. Lorand, ed), Academic Press, New York, 1979, vol. 80, pp. 341-361.
A4. Solution composition dependent variation in extinction coefficients for p-nitroaniline. R. Lottenberg and C.M. Jackson, Biochimica et Biophysica Acta, 742 (1983) 558-564.
A5. A rapid procedure for the large scale purification of elastase and cathepsin G from human sputum. R.R. Martodam, R.J. Baugh, D.Y. Twumasi and I.E. Liener, Preparative Biochemistry, 9 (1979) 15-31.
A6. The determination of enzyme inhibitor constants. M. Dixon, The Biochemical Journal, 55 (1953) 170-171.
A7. The kinetics of reversible tight-binding inhibition. J.W.Williams and J.F. Morrison, in Methods in Enzymology (D.L. Purich, ed), Academic Press, New York, 1979, vol. 63, pp. 437-467.
A8. Two convenient spectrophotometric enzyme assays. A biochemistry experiment in kinetics. J.A. Hurlbut, T.N. Ball, H.C. Pound and J.L. Graves, Journal of Chemical Education, 50 (1973) 149-151.
A9. The preparation and properties of two new chromogenic substrates of trypsin. B.F. Erlanger, N. Kokowsky and W. Cohen, Archives of Biochemistry and Biophysics, 95 (1961) 271-278.
A10. The human complement system serine proteases Clr and Cls and their proenzymes. R.B. Sim, in Methods in Enzymology (L. Lorand, ed), Academic Press, New York, 1979, vol.80, pp 26-42.
A11. Extrinsic plasminogen activator and urokinase. J.H. Verheijen, C. Kluft, G.T.G. Chang and E. Mullaart, in Methods of Enzymatic Analysis (H.U. Bergmeyer, J. Bergmeyer and M. Grassl, eds), Verlag Chemie, Weinheim, 1984, third edition, vol. 5, pp. 425-433.
A12. Sperm acrosin. W. Mueller-Esterl and H. Fritz, in Methods in Enzymology (L. Lorand, ed), Academic Press, New York, 1979, vol. 80, pp. 621-632.
A13. Novel method for detection of betalactamases by using a chromogenic cephalosporin substrate. C.H. O'Callaghan, A. Morris, S.M. Kirby and A.H. Shingler, Antimicrobial Agents and Chemotherapy, 1 (1972) 283-288.
A14. Cathepsin B, cathepsin H, and cathepsin L. A.J. Barrett and H. Kirschke, in Methods in Enzymology (L. Lorand, ed), Academic Press, New York, 1979, vol. 80, pp. 535-561.
A15. Pepsins, gastricsins and their zymogens. A.P. Ryle, in Method of Enzymatic Analysis (H.U. Bergmeyer, J. Bergmeyer and M. Grassl, eds), Verlag Chemie, Weinheim, 1984, third edition, vol. 5, pp. 223-238.
A16. Cathepsin D, cathepsin E. V. Turk, T. Lah and I. Kregar, in Methods of Enzymatic Analysis (H.U. Bergmeyer, J. Bergmeyer and M. Grassl, eds), Verlag Chemie, Weinheim, 1984, third edition, vol 5, pp. 211-222.
A17. Amino acid arylamidase. J.C.M. Hafkenscheid, in Methods of Enzymatic Analysis (H.U. Bergmeyer, J. Bergmeyer and M. Grassl. eds), Verlag Chemie, Weinheim, 1984, third edition, vol. 5, pp. 11-15.
A18. Angiotensin I converting enzyme (kininase II). J.W. Ryan, in Methods of Enzymatic Analysis (H.U. Bergmeyer, J. Bergmeyer and M. Grassl. eds), Verlag Chemie, Weinheim, 1984, third edition, vol. 5, pp. 20-34.
A19. Renin. T.Inagami and M. Naruse, in Methods of Enzymatic Analysis (H.U. Bergmeyer, J. Bergmeyer and M. Grassl, eds), Verlag Chemie, Weinheim, 1984, third edition, vol. 5, pp. 249-258.
A20. The third, fourth, and fifth components of human complement: isolation and biochemical properties. B.F. Tack, J. Janatova, M.L. Thomas, R.A. Harrison and C.H. Hammer, in Methods in Enzymology (L. Lorand, ed), Academic Press, new York, 1979, vol. 870, pp. 64-101.

By following the techniques referenced above, as well as by utilization of other known techniques, as well as by comparison with compounds known to be useful for treatment of the above-mentioned disease states, it is believed that adequate material is available to enable one of ordinary skill in the art to practice the invention. Of course, in the end-use application of the compounds of this invention, the compounds are preferably formulated into suitable pharmaceutical preparations such as tablets, capsules or elixers, for oral administration or in sterile solutions or suspensions for parenteral administration. The compounds of this invention can be administered to patients (animals and human) in need of such treatment in a dosage range of 5 to 500 mg per patient generally given several times, thus giving a total daily dose of from 5 to 2000 mg per day. As stated above, the dose will vary depending on severity of disease, weight of patient and other factors which a person skilled in the art will recognize.

Typically the compounds described above are formulated into pharmaceutical compositions as discussed below.

About 10 to 500 mg of a compound or mixture of compounds of formula I or a physiologically acceptable salt is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type, a liquid carrier such as fatty oil. Various other materials may he present as coatings or to otherwise modify the physical form of the dosage unit. For instance, tablets may be coated with shellac, sugar or both. A syrup or elixer may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

While the invention has been described in connection with specific embodiments thereof, it will be understood that it is capable of further modifications and this application is intended to cover any variations, uses, or adaptations of the invention following, In general, the principles of the invention and including such departures from the present disclosure as come within known or customary practice within the art to which the invention pertains and as may be applied to the essential features hereinbefore set forth, and as follows in the scope of the appended claims.

## Claims

1. A compound of the formula
R₁NHCHR₂COCF₂CHR₃(NR_{b}COXRₐ)ₙQ
the hydrates, isosteres or the pharmaceutically acceptable salts thereof wherein
Ri is hydrogen, an amino protecting group selected from Group K, an a-amino acid or a peptide comprised of a number of a-amino acid building blocks, each of said a-amino acid or peptide optionally bearing an amino protecting group preferably selected from Group K,
R₂ is a specific characteristic "R group" side chain of the a-amino acid building block responsible for directing the inhibitor to the active site of the enzyme,
R₃ is H, C₁₋₆ straight or branched alkyl, phenyl, benzyl, cyclohexyl, cyclohexylmethyl or 2-pyridylmethyl, or is the R-group residue of an a-amino acid building block for that peptidase substrate analog,
n is an integer of 1 to 10,
Rₐ is a specific characteristic "R-group" side chain of an a-amino acid building block for that peptidase substrate analog, or is an ethylene moiety which when attached to the N atom of that retroamide moiety forms a 2-oxopyrrolidine moiety,
R_{b} is H, C₁₋₆ straight or branched alkyl, or an ethylene moiety which when linked to the CH moiety of X forms a 2-oxopyrrolidine moiety,
X is H, CH, OR₇ or R₇ with R₇ being C₁₋₆ straight or branched alkyl, phenyl, benzyl, cyclohexyl, cyclohexylmethyl or 2-pyridylmethyl with the proviso that when X is other than CH, Rₐ and Q are deleted, Q is H, C₁₋₁₀ alkyl or arylalkyl, CORsY, or COY with Rs being an a-amino acid R-group or a peptide comprised of a-amino acid building blocks,
Y is NHR₄ or OR₄, with R₄ being H, Ci-e straight or branched alkyl, phenyl, benzyl, cyclohexyl, cyclohexylmethyl or 2-pyridylmethyl, wherein the said a-amino acid and peptide moieties are bulding blocks selected from Groups A. B, C, D, E, F, G and J, and K is a terminal amino protecting group, members of these groups being
Group A: Lys and Arg
B: Ser, Thr, Gin, Asn, Cys, His, and N-methyl derivatives
D: Pro, Ind
E: Ala, Leu, Ile, Val, n-Val, Met, n-Leu and N-methyl derivatives
F: Phe, Tyr, O-methyl Tyrosine, Trp, Nal(1), and N-methyl derivatives
G: Gly, Sar
J: 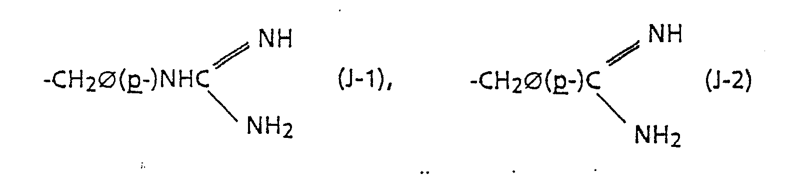 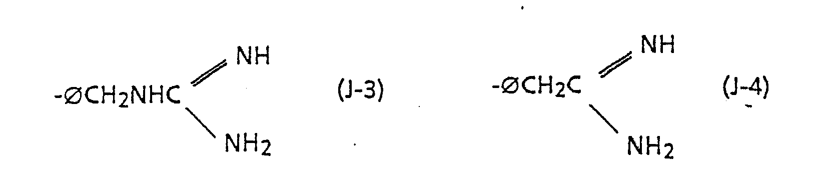 with 0, of course, representing phenyl (it being understood that the bond of J1-4 is always attached to an amino acid),
K: Acetyl (Ac), Succinyl (Suc), Benzoyl (Bz), t-Butyloxycarbonyl (Boc), Carbobenzoxy (CBZ), Tosyl (Ts), Dansyl (DNS), Isovaleryl (Iva), Methoxysucclnyl (MeOSuc), 1-Adamantanesulphonyl (AdS0₂), 1-Adamantaneacetyl (AdAc), 2-Carboxybenzoyl (2-CBZ), phenylacetyl (cobenzyl), tert-butylacetyl (Tba) and bis[1-naphthyl)methyl]acetyl (BNMA) and such other terminal amino protecting groups which are functionally equivalent thereto.

2. A compound of claim 1 useful for inhibiting human leukocyte elastase wherein
R₁ is -P₂P₃P₄ with
P₂ being a-amino acid block having an R group selected from Groups D, E and F,
P₃ is the a-amino acid blocks of Group D, E, or lysine,
P₄ is the a-amino acid blocks of Groups E or zero;
R₂ is the R group of Groups E and G,
Ra is an R group selected from Groups E and G,
Rₐ is an R Group selected from Groups E and G, and
R_{b}, X, n and Q are as generically defined for formula I.

3. A compound of claim 2 selected from the group consisting of

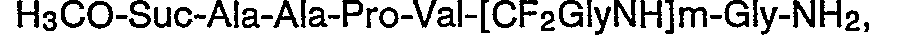

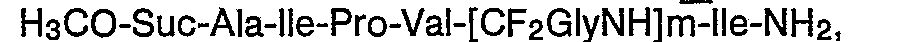

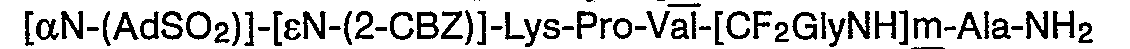

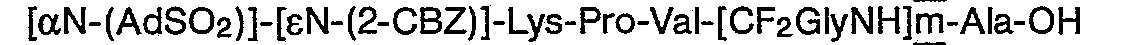

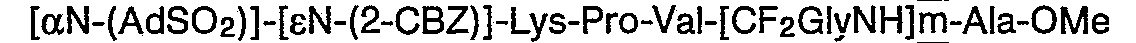

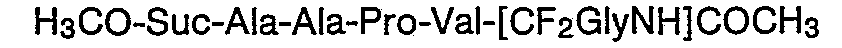

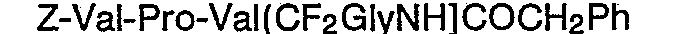

4. A compound of claim 1 useful for inhibiting Cathepsin G wherein
Ri is -P₂P₃P₄P₅ with
P₂ being selected from Groups D, E, or G,
P₃ is selected from Groups E or G,
P₄ is selected from Groups E, G or is deleted,
R₂ is selected from Groups E and F,
R₃, Ra, R_{b}, X, n and Q are as generically defined in claim 1.

5. A compound of claim 4 selected from the group consisting of

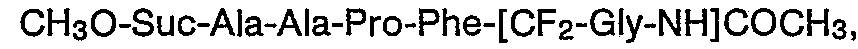

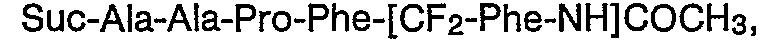

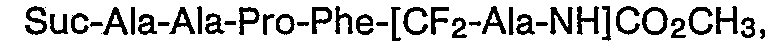

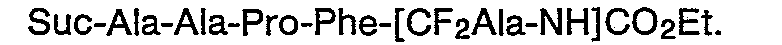

6. A compound of claim 1 useful for inhibiting thrombin wherein R₁ is (a)-P₂-P₃, (b) -P₂ or (c) -P₂-P₃-P₄ wherein
(a) P₂ is selected from Groups D, E or F,
P₃ is selected from Group F, each P₃ being in the D-configuration,
(b) P₂ is selected from Group K,
(c) P₂ is selected from Group E,
P₃ is selected from Groups C, G and E,
P₄ is selected from Groups F, G and E or is zero,
R₂ is the arginine side chain, or is selected from Groups A and J,
R₃ is as generically defined formed for formula I, with the residue being selected from Groups C and G,
Rₐ is selected from Group C or G,
R_{b}, n, X and Q are as generically defined for formula I, and
Rs is H or is a residue of an a-amino acid of Group E or D or is zero.

7. A compound of claim 6 selected from the group consisting of 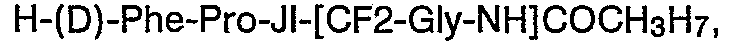 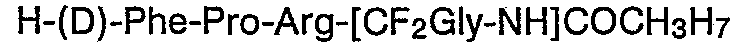 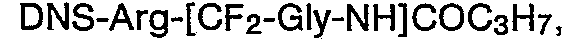 and

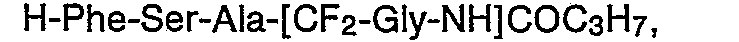

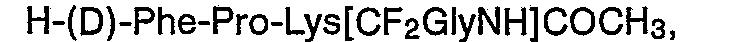

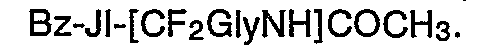

8. A compound of claim 1 useful for inhibiting chymotrypsin wherein
Rs, Ra, R_{b}, X and Q are defined for compounds of claim 1 and
R₁ is -P₂P₃P₄Ps with
P₂ being selected from Groups D, E, G or K,
P₃ is selected from Groups E or G or K or is deleted,
P₄ is selected from Groups E or G or K or is deleted,
P₅ is selected from Group K or is deleted, and R₂ is selected from Groups E and F.

9. A compound of claim 8 selected from the group consisting of 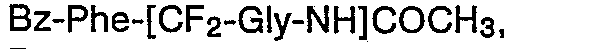 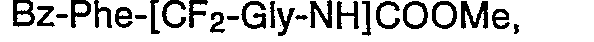 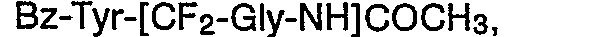 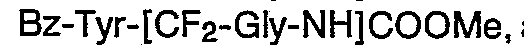 and

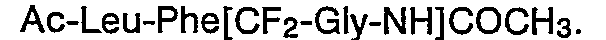

10. A compound of claim 1 useful for inhibiting trypsin wherein
R₂, R₃, Ra, R_{b}, n, X and Q generically are as defined in claim 1,
Ri is selected from (a)-P₂-P₃, (b)-P₂ or (c)-P₂-P₃-P4 with
(a) P₂ is selected from Groups E or F, Ps is selected from from Group F, (each being in the D-configuration),
(b) P₂ is selected from Group K,
(c) P₂ is selected from Group D or E, P₃ is selected from Groups G and E, P₄ is selected from Groups G and E or is zero.

11. A compound of claim 8 useful for inhibiting plasmin wherein
Rs, Rₐ, R_{b}, X, n and Q are as defined in claim 1,
R₁ is -P₂P₃P₄ with
P₂ being selected from Group E or F,
Ps is selected from Groups B, F or K, and
P₄ is selected from Group K,
R₂ is selected from Groups A and J.

12. A compound of claim 11 selected from the group consisting of 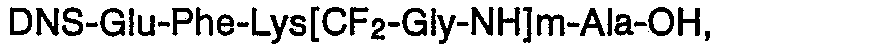 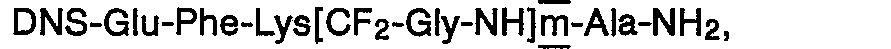 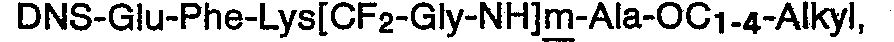 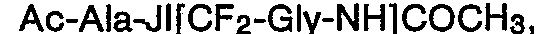 and

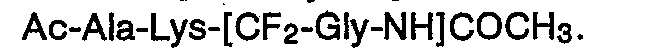

13. A compound of claim 1 useful for inhibiting Ci-esterase wherein
Q, R₃, R_{b} and X are defined in claim 1, with the R-group of Rs selected from Group E or G,
R₁ generically is -P₂-P₃ with
P₂ being selected from Groups E, G, D, C, F, A or B,
P₃ is selected from Group K,
R₂ is selected from Groups A and J,
R₃ is selected from Group E or G.

14. A compound of claim 13 selected from the group consisting of

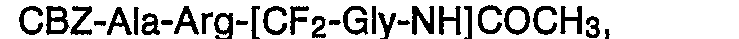

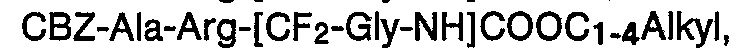

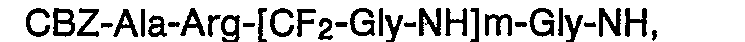

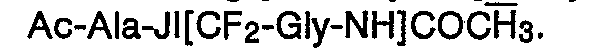

15. A compound of claim 1 useful for Inhibiting Cs-convertase wherein
R_{b}, X, n and Q are as defined in claim 1,
R₁ is -P₂P₃P₄ with
P₂ being selected from Groups E or F,
P₃ is selected from Groups E or F, and
P₄ is selected from Group K,
R₂ is selected from Groups A or J,
R₃ is selected from Groups E or G,
Rs is zero, and
Ra is selected from Group E.

16. A compound of claim 15 selected from the group consisting of

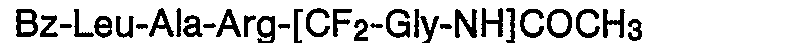

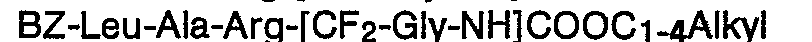

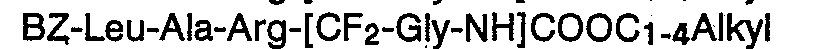

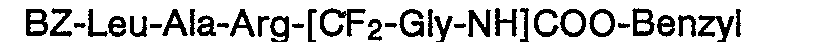

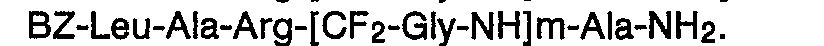

17. A compound of claim 1 useful for inhibiting urokinase wherein
R₃, Rₐ, R_{b}, X, n and Q are as defined in claim 1,
R is -P₂P₃ with
P₂ being selected from Groups E and G, and
P₃ is selected from Group B,
R₂ is selected from Groups A and J, and the R-group of R₃ and
Ra being selected from Group E.

18. A compound of claim 17 selected from the group consisting of

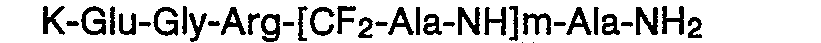

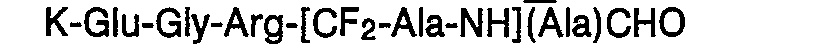

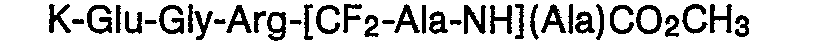

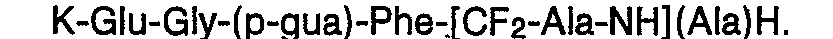

19. A compound of claim 1 useful for inhibiting plasminogen activator wherein R3, Ra, Rb, X, n and Q are as defined in claim 1,
R1 generically is -P2P3P4 wherein
P2 is Gly,
P3 is selected from Group B, and
P4 is selected from Group K, and
R2 is selected from Groups A and J.

20. A compound of claim 19 selected from the group consisting of

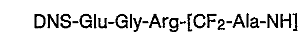

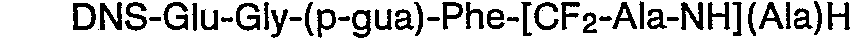

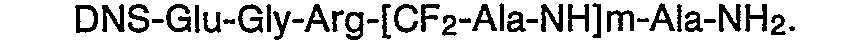

21. A compound of claim 1 useful for inhibiting acrosin wherein
Rs, Ra, R_{b}, X, n and Q are as defined in claim 1,
R₁ is -P₂P₃P₄ with
P₂ being selected from Group E or K,
P₃ is selected from Group E or is deleted,
P₄ is selected from Group K or is deleted,
R₂ is selected from Groups A and J, the R-groups of
Rₐ being selected from Group E or G.

22. A compound of claim 21 selected from the group consisting of 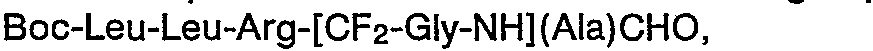 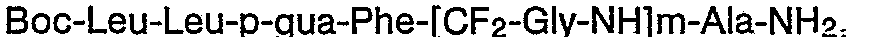 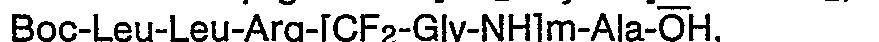 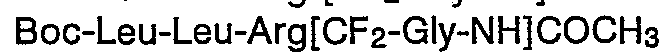 and

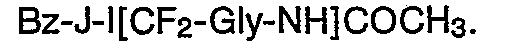

23. A compound of claim 1 useful for inhibiting β-lactamase wherein
R₁ is P₂,
P₂ being selected from Group K,
R₂ is selected from Groups E, G and C, and
R₃, Rₐ, R_{b}, X, n and Q are as defined in claim 1 with the R group of R₃ being selected from Group E or G with the proviso that the P₂ carbonyl moiety may also exist in its chemically reduced form.

24. A compound of claim 23 selected from the group consisting of

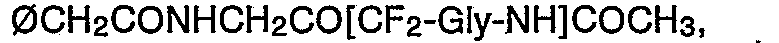

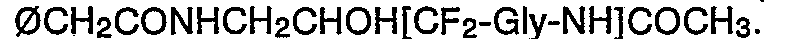

25. A compound of claim 1 useful for inhibiting D-Ala-D-Ala carboxypeptidase wherein
Ri is P₂-P₃ with
P₂ being Ns-Ac-Lys or is selected from Groups E and C,
P₃ is selected from Group K,
R₂ is D-ala, and
R₃, Ra, R_{b},X and Q are as defined in claim 1, with the R-group of R₃ being selected from Group E.

26. A compound of claim 25 selected from group consisting of

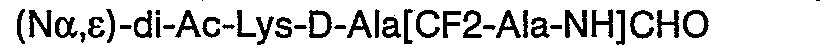

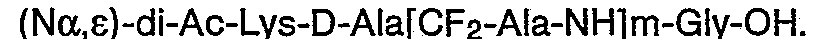

27. A compound of claim 1 useful for inhibiting Cathepsin B wherein
Ri is (a) -P2-P₃ or (b) -P₂-P₃-P₄ wherein
(a) P₂ is selected from Groups E and F,
P₃ is selected from Group K,
(b) P₂ is selected from Groups E and F,
P₃ being selected from Groups E and F,
P₄ is selected from Group K,
R₂ is selected from Groups A and J or ThrOCH₂0, and R₃, Rₐ, R_{b}, X, n and Q are as defined in claim 1.

28. A compound of claim 1 selected from the group consisting of

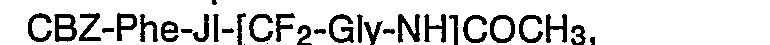

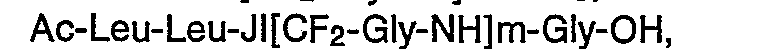

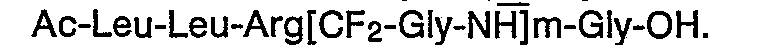

29. A compound of claim 1 useful for inhibiting renin wherein
Ri is -P₂P₃P₄P₅P₆ wherein
P₂ is selected from Groups E, C, or F,
P₃ is selected from Groups E or F or is deleted,
P₄ is selected from Groups E, D, F or is deleted,
Ps is selected from Groups E, C, F or is deleted,
P₆ is selected from Group K,
R₂ is selected from Groups E or F or is cyclohexylmethylene,
Rs, Rₐ, R_{b}, X, n and Q are as defined in claim 1 with Rₐ preferably being selected from Groups G, E, or F, Rs, when present, is (a) Pₓ' or (b) Pₓ'-Pₓ'+1,
Px' being selected from Groups E, C or F,
Px'+1 being selected from Groups E or C or is Lys,
with the proviso that the P' carbonyl moiety may also be in its chemically reduced form.

30. A compound of claim 29 selected from the group consisting of

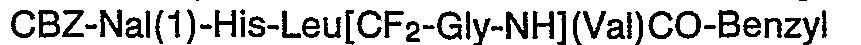

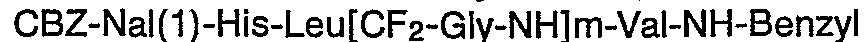

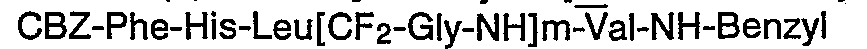

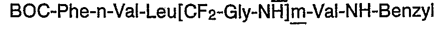

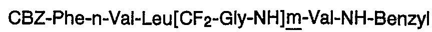

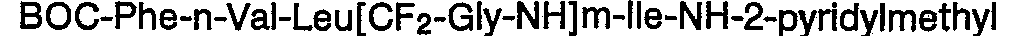

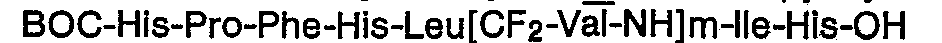

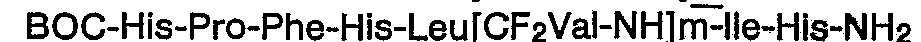

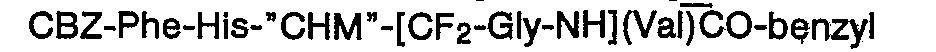

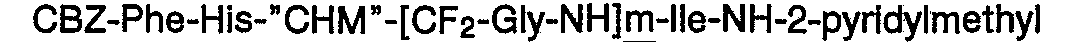

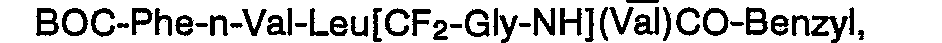

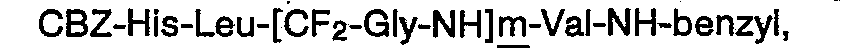

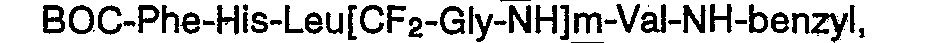

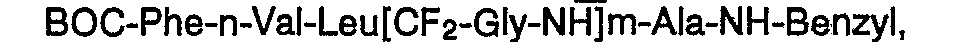

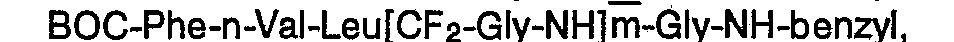

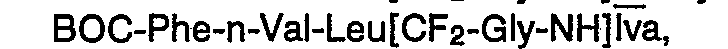

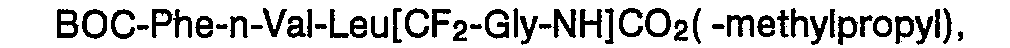

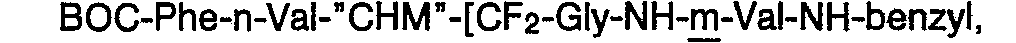

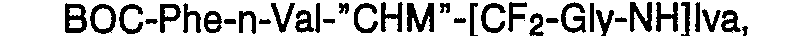

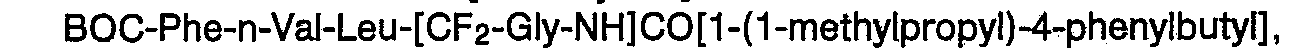

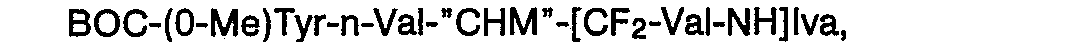

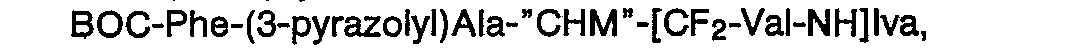

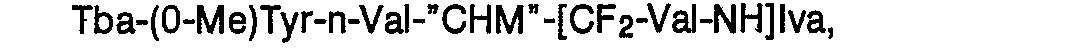

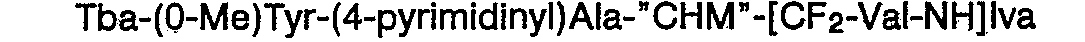

31. A compound of claim 1 useful for inhibiting pepsin wherein
R₃, Ra, Rb, X, n and Q are as defined in claim 1,
R₁ is -P₂P₃P₄ with
P2 being selected from Groups E or F,
P3 being selected from Groups E or F,
P4 is selected from Group K,
R₂ is selected from Groups E and F.

32. A compound of claim 31 selected from the group consisting of

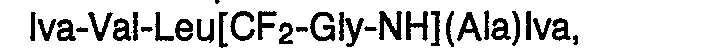

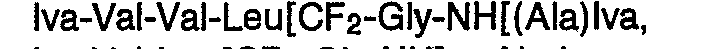

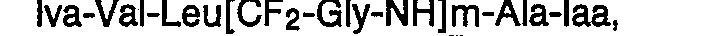

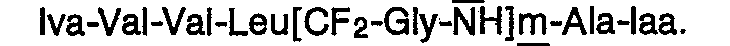

33. A compound of claim 1 useful for inhibiting Cathepsin D wherein
R₃, Rₐ, R_{b}, n, X and Q are as defined in claim 1,
R₁ generically is -P₂PsP4 with
P₂ being selected from Groups E and F,
P₃ is selected from Groups E and F or is deleted,
P₄ is selected from Group K,
R₂ is selected from Groups E and F.

34. A compound of claim 33 selected from the group consisting of

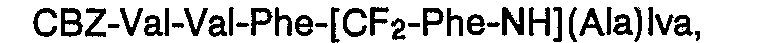

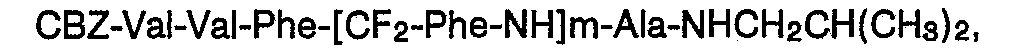

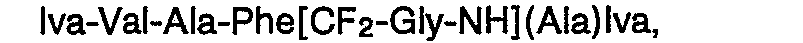

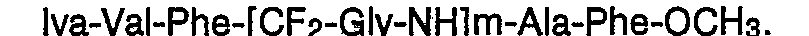

35. A compound of claim 1 useful for inhibiting angiotensin converting enzyme wherein Rs, Ra, R_{b}, X, n and Q are as defined in claim 1,
R₁ is selected from Group K,
R₂ is selected from Groups E, F and G.

36. A compound of claim 35 selected from the group consisting of

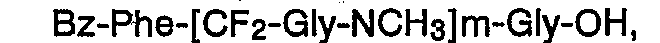

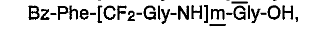

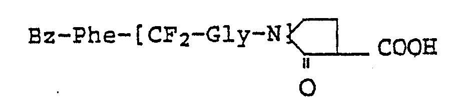

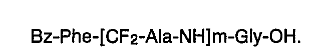

37. A compound of claim 1 useful for inhibiting enkephalinase wherein Rs, Rₐ, R_{b}, n and Q are as defined in claim 1,
R₁ is -P₂P₃ with
P₂ being Gly and
Ps being selected from Group F or Is deleted,
R₂ is Gly.

38. A comoound of claim 1 selected from the group consistina of

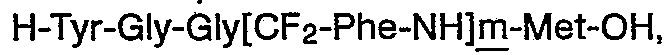

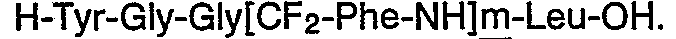

39. A compound of claim 1 useful for inhibiting pseudomonas elastase wherein
Rs, Rₐ, R_{b}, X, n and Q are as defined in claim 1,
R₁ is -P₂P₃ with
P₂ being selected from Group E,
P₃ is selected from Group K,
R₂ is selected from Groups E and G.

40. A compound of claim 39, said compound being MeOSUC-Ala-Ala-[CF₂-lle-NH]m-Ala-NH₂.

41. A compound of claim 1 useful for inhibiting leucine amino peptidase wherein
Rs, Rₐ, R_{b}, X, n and Q are as defined in claim 1,
Ri is hydrogen, and
R₂ is selected from Groups A, B, E, F and J.

42. A compound of claim 41 selected from the group consisting of

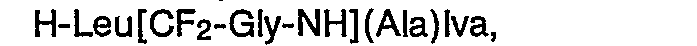

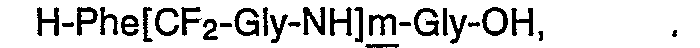

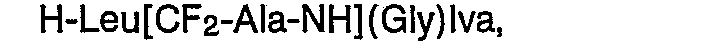

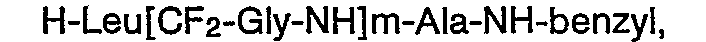

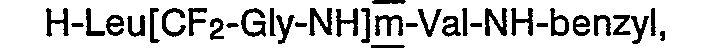

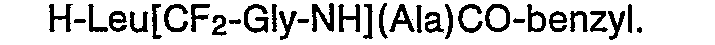

43. A compound of claim 1 useful for inhibiting kallikreins wherein
R₃, Rₐ, R_{b} X, n and Q are as defined in claim 1,R₁ is -P₂P₃ with
P₂ being selected from Groups E and F,
Ps being selected from Groups C, E or F, the residuesof which may be in either the D- or L-configuration, and
R₂ is selected from Groups A orT.

44. A compound of claim 43 selected from the group consisting of

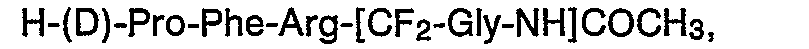

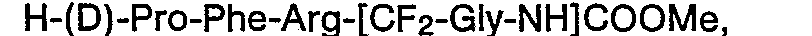

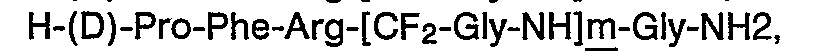

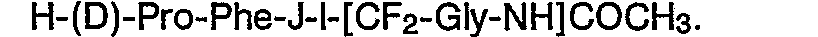

45. Compounds of the Formula 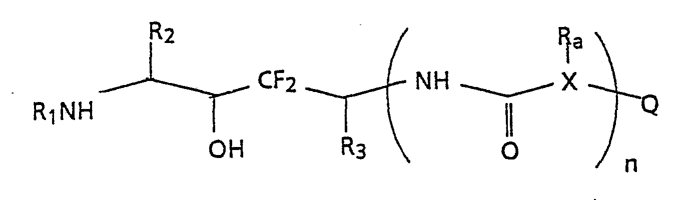 wherein R₁, R₂, R₃, 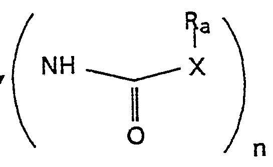 and Q are as defined in Claim 1.

46. A process for preparing a compound of the formula 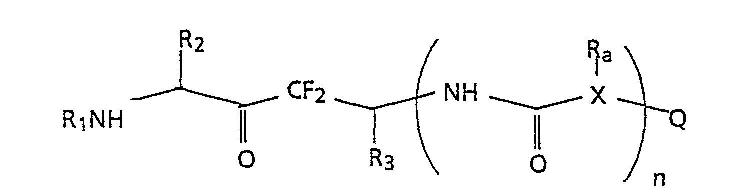 wherein R1, R2, R3, X, Ra, n and Q are as defined in Claim 1, which comprises oxidizing a compound of the Formula 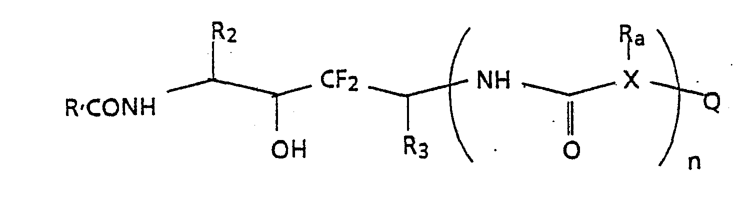 according to oxidation procedures (a), (b), (c) or (d) as follows:
a) preparing an In situ sulfonium adduct by reacting about 2 to 6 equivalents of dimethylsulfoxide with about 1 to 3 equivalents of (CF₃CO)₂O or (COCI)₂; said reactants being dissolved in an inert solvent, under anhydrous conditions within the temperature range of about -80°C to -50°C, contacting said sulfonium adduct with about 1 equivalent of an alcohol of formula II, said alcohol being dissolved in an inert solvent or minimum amounts of dimethylsulfoxide, allowing the reactants to react at about -50°C for about 10 to 30 minutes, and completing the reaction by the addition of about 3 to 10 equivalents of a tertiary amine;
b) reacting an alcohol of formula II with pyridinium dichromate by contacting the reactants together In a powdered water-trapping molecular sieve In the presence of glacial acetic acid at about 0° C to 50°C;
c) reacting an alcohol of formula II with about 1 to 5 equivalents of a chromic anhydrldepyridine complex, said complex being formed In situ in an inert solvent under an inert atmosphere using anhydrous conditions, said reaction of the alcohol being effected in the chromic anhydride-pyridine complex-reaction mixture for about 1 to 15 hours;
d) reacting an alcohol of formula II with 1,1,1-triacetoxy-2,1-benzoxiodol-3(3H)-one, said reaction being effected In an inert solvent under anhydrous conditions in an inert atmosphere for about 1 to 48 hours, reactions a, b, c or d being followed by an optional deprotection of any protected amine.
